(19)

Europäisches
Patentamt
European
Patent Office
Office européen
des brevets

(11)    **EP 3 054 300 A1**

(12)    # EUROPEAN PATENT APPLICATION
published in accordance with Art. 153(4) EPC

(43) Date of publication:
**10.08.2016 Bulletin 2016/32**

(21) Application number: **14832504.6**

(22) Date of filing: **01.08.2014**

(51) Int Cl.:
***G01N 33/68*** (2006.01)

(86) International application number:
**PCT/ES2014/070634**

(87) International publication number:
**WO 2015/015043 (05.02.2015 Gazette 2015/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.08.2013 ES 201331200**

(71) Applicants:
• **Fundación Para La Investigación Biomédica Del Hospital Universitario Ramón Y Cajal**
**28034 Madrid (ES)**
• **Fundación Para La Investigación Biomédica Del Hospital Clínico San Carlos**
**28040 Madrid (ES)**

(72) Inventors:
• **DE LA HOZ CABALLER, Ma Belén**
**E-28034 Madrid (ES)**
• **MARTÍNEZ BOTAS, Javier**
**E-28034 Madrid (ES)**
• **CERECEDO CARBALLO, Inmaculada**
**E-28034 Madrid (ES)**
• **FERNÁNDEZ RIVAS, Montserrat**
**E-28040 Madrid (ES)**
• **RODRÍGUEZ ÁLVAREZ, Mónica**
**E-28040 Madrid (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54)    **METHOD FOR PROGNOSIS OF THE EFFICACY OF ORAL IMMUNOTHERAPY FOR THE TREATMENT OF ALLERGY TO PROTEINS IN COW'S MILK**

(57)    The present invention relates to a method for prognosis of the efficacy of oral immunotherapy for the treatment of allergy to proteins in cow's milk providing a solution to the problems stated in the state of the art since it provides a method which allows making a prognosis of the number of reactions that will be produced during oral immunotherapy (OIT) against proteins in cow's milk and/or an estimate of the treatment time required to achieve tolerance or desensitization during OIT against proteins in cow's milk and/or the need for pre-medication during OIT in a human subject.

**EP 3 054 300 A1**

**Description**

**FIELD OF THE INVENTION**

The present invention is comprised in the general field of allergology and relates particularly to a method for prognosis of the efficacy of the treatment for desensitization to cow's milk.

**PRIOR ART**

Food allergy with immediate hypersensitivity does not have an established effective treatment today, the only alternative for the patients is to avoid same until spontaneous tolerance to said food is achieved, a situation achieved by a large number of patients but not by all.

Allergy to proteins in cow's milk (APCW) occurs when an individual, after consuming dairy products, shows an abnormal response (with symptoms that can be included in adverse reactions to food) and when there is a proven immunological mechanism in that process.

Proteins responsible for allergenicity in cow's milk are caseins (alpha-casein, beta-casein and kappa-casein) and beta-lactoglobulin, alpha-lactalbumin, bovine serum albumin and bovine immunoglobulins serum proteins as well as other proteins at a lower proportion: lactoferrin, transferrin, lipase which are serum proteins and represent 2% of the total protein in whole cow's milk.

Caseins are the main cause of allergy to cow's milk.

It has recently been proven that there are different clinical phenotypes of reaction severity in food allergy. Although several factors have been involved in these differences, the geographical component plays a very significant role. A clear example is fruit allergy in which individuals who are allergic to a certain food because they recognize different allergens (whole protein/peptide) have different clinical severity. Therefore, individuals who are allergic to peaches from the north of Europe only have mild symptoms after consuming them, while patients from the south of Europe have severe systemic reactions. Knowing the recognition pattern of allergens of a specific food, both the whole proteins and ideally the epitopes thereof, which a specific population has, is therefore of greatest interest.

There are different methods today for diagnosing allergy to cow's milk, but as a norm, detection of IgE specific to different proteins in cow's milk is performed after the first allergic attack.

Oral immunotherapy (OIT) with food is a novel therapeutic method the objective of which is to achieve in patients allergic to a specific food tolerance to same by means of the continuous administration of said food starting from minimal doses until reaching tolerance to a common amount of the food.

Cow's milk is a ubiquitous food that is very hard to avoid and produces severe reactions. In the last ten years, many research groups have developed protocols for the oral administration of cow's milk in order to achieve tolerance to same. In the current state of knowledge, the treatment achieves good efficacy levels as it successfully induces tolerance to cow's milk. Nevertheless, its biggest problem lies in the safety of the treatment since the patients show a high number of allergic reactions when performing the procedure. Although said allergic reactions are mild and easily treatable on most occasions, they are severe and even require the administration of adrenaline on very few occasions. Therefore, reactions during the procedure are one of the main difficulties when it comes to incorporating the procedure into a common practice for the disease, and therefore expanding the use thereof to a significant number of patients.

There is therefore a need to provide a method for prognosis of the efficacy of the treatment for desensitization to cow's milk and therefore of the patient response to same.

**DISCLOSURE OF THE INVENTION**

The present invention provides a solution to the problems stated in the state of the art since it provides a method which allows making a prognosis of the number of reactions that will be produced during oral immunotherapy (OIT) against proteins in cow's milk and/or an estimate of the treatment time required to achieve tolerance or desensitization during OIT against proteins in cow's milk and/or the need for pre-medication during OIT in a human subject.

Therefore, a first aspect of the invention relates to a method for prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT in a human subject, comprising the following steps:

a. Isolating a biological sample from the subject;
b. Determining the presence or absence of IgE antibodies in the biological sample of step a) against or with specificity for peptides identified with the following sequences:

a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and/or

b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and/or

c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and/or

d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and/or

e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active;

where the prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment.

A preferred embodiment of the first aspect of the invention relates to a method for prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT in a human subject, comprising the following steps:

a. Isolating a biological sample from the subject;

b. Determining the presence or absence of IgE antibodies in the biological sample of step a) against or with specificity for peptides identified with the following sequences:

a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and

b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and

c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and optionally

d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and optionally

e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active;

where the prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment.

Another preferred embodiment of the first aspect of the invention relates to a method for prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT in a human subject, comprising the following steps:

a. Isolating a biological sample from the subject;
b. Determining the presence or absence of IgE antibodies in the biological sample of step a) against or with specificity for peptides identified with the following sequences:

a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and
b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and
c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and
d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and
e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active;

where the prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment.

In another preferred embodiment of the first aspect of the invention, the method is a method for prognosis of the number of reactions during oral immunotherapy (OIT) and the determination of the presence or absence of IgE antibodies of step b) in the biological sample of step a) is performed by determining the presence or absence of IgE antibodies against or with specificity for at least the following peptides identified with the following sequences:

a. sequences SEQ ID NO: 19, 21, 25 and 27 and/or derivatives of sequences SEQ ID NO: 19, 21, 25 and 27 that are immunologically active; and
b. sequences SEQ ID NO: 36, 37 and 38 and/or derivatives of sequences SEQ ID NO: 36, 37 and 38 that are immunologically active; and
c. sequences SEQ ID NO: 51, 53, 58, 59 and 62 and/or derivatives of sequences SEQ ID NO: 51, 53, 58, 59 and 62 that are immunologically active; and
d. sequence SEQ ID NO: 69 and/or a derivative of the sequence SEQ ID NO: 69 that is immunologically active; and
e. sequences SEQ ID NO: 71 and 72 and/or derivatives of sequences SEQ ID NO: 71 and 72 that are immunologically active;

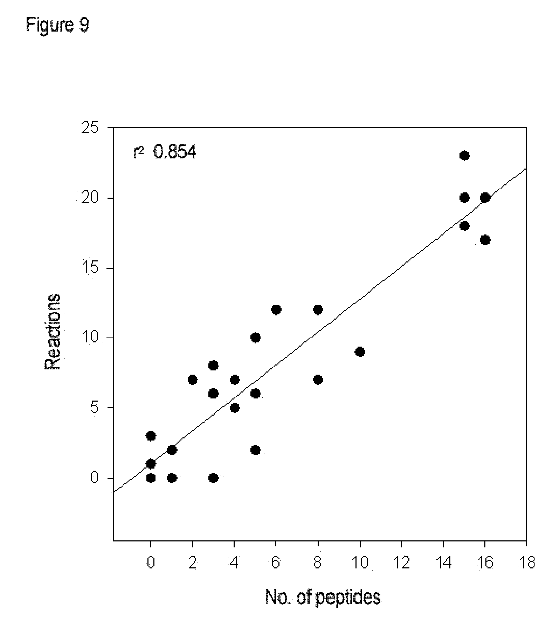

where the prognosis of the number of reactions during oral immunotherapy (OIT) against proteins in cow's milk is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment. Preferably, the reference values are established in Figure 9.

In another preferred embodiment of the first aspect of the invention, the method is a method for prognosis of an estimate of the treatment time required to achieve tolerance or desensitization during OIT and the determination of the presence or absence of IgE antibodies of step b) in the biological sample of step a) is performed by determining the presence or absence of IgE antibodies against or with specificity for at least the following peptides identified with the following sequences:

a. sequences SEQ ID NO: 28 and 31 and/or derivatives of sequences SEQ ID NO: 28 and 31 that are immunologically active; and
b. sequences SEQ ID NO: 36-40 and 44 and/or derivatives of sequences SEQ ID NO: 36-40 and 44 that are immunologically active; and

c. sequences SEQ ID NO: 53, 55 and 56 and/or derivatives of sequences SEQ ID NO: 53, 55 and 56 that are immunologically active; and

d. sequences SEQ ID NO: 67 and 68 and/or a derivative of the sequence SEQ ID NO: 67 and 68 that are immunologically active; and

e. sequences SEQ ID NO: 70, 72 and 75 and/or derivatives of sequences SEQ ID NO: 70, 72 and 75 that are immunologically active;

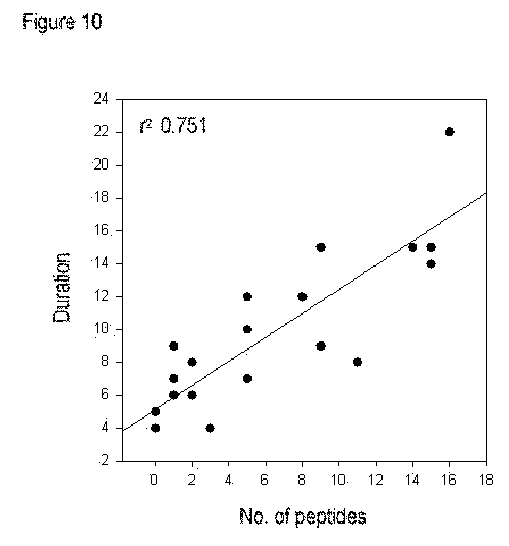

where an estimate of the treatment time required to achieve tolerance or desensitization during OIT against proteins in cow's milk is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment. Preferably, the reference values are established in Figure 10.

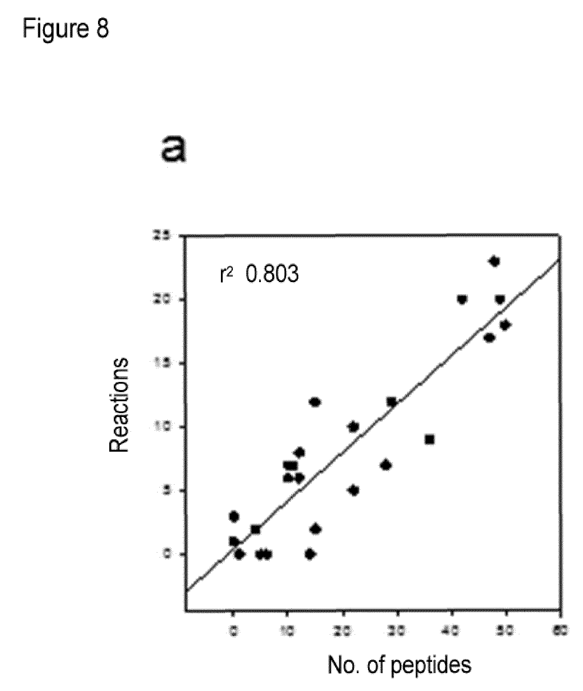

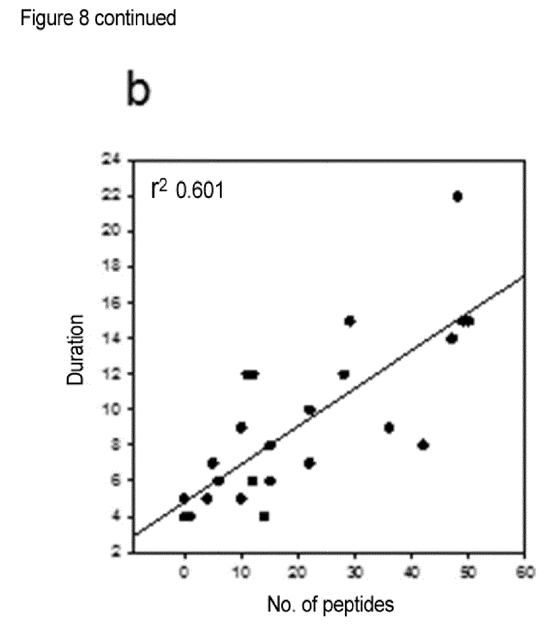

In yet another preferred embodiment of the first aspect of the invention, the determination of the presence or absence of IgE antibodies in the biological sample of step a) is performed by determining the presence or absence of IgE antibodies against or with specificity for at least the following peptides identified with the following sequences: SEQ ID NO: 18-75 and/or derivatives of sequences SEQ ID NO: 18-75 that are immunologically active, where the prognosis of the number of reactions during oral immunotherapy (OIT) against proteins in cow's milk is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment. Preferably, the reference values are established in Figure 8.

In yet another preferred embodiment of the first aspect of the invention, the determination of the presence or absence of IgE antibodies in the biological sample of step a) is performed by determining the presence or absence of IgE antibodies against or with specificity for at least the following peptides identified with the following sequences: SEQ ID NO: 18-75 and/or preferably derivatives of sequences SEQ ID NO: 18-75 that are immunologically active, where:

- a peptide recognition of less than 15% is indicative of a good prognosis,
- a peptide recognition of between 15%-75% is indicative of a moderate prognosis
- a peptide recognition of more than 75% is indicative of a poor prognosis.

In a preferred embodiment of the first aspect of the invention or of any of the preferred embodiments thereof, the biological sample includes different types of tissue samples, as well as biological fluid samples, such as blood, serum, plasma, cerebrospinal fluid, peritoneal fluid, feces or urine. The sample is preferably selected from blood, plasma, serum or urine.

In a preferred embodiment of the first aspect of the invention or of any of the preferred embodiments thereof, the presence of peptides is determined by means of an immunoassay.

In a preferred embodiment of the first aspect of the invention or of any of the preferred embodiments thereof, the presence of peptides is determined by means of peptide microarrays.

A second aspect of the invention relates to a peptide microarray suitable for carrying out the method of the first aspect of the invention or any of the preferred embodiments thereof, comprising the combinations of peptide sequences defined in said method or in said embodiments.

A third aspect relates to a composition suitable for carrying out the method of the first aspect of the invention or any of the preferred embodiments thereof, comprising the combinations of peptides defined in said method or in said embodiments.

A fourth aspect relates to a kit suitable for carrying out the method of the first aspect of the invention or any of the preferred embodiments thereof, comprising the combinations of peptides defined in said method or in said embodiments.

In a preferred embodiment of the fourth aspect of the invention, said kit comprises:

a. Recognition molecules (capture biomolecules) capable of recognizing IgE selected from the list consisting of the different combinations of peptide sequences defined in the method of the first aspect of the invention or in any of the preferred embodiments thereof; and

b. A second recognition molecule (detection biomolecule) capable of recognizing the target analyte or the capture biomolecule optionally bound to a tag molecule.

In another preferred embodiment of the fourth aspect of the invention, said kit comprises:

a. Recognition molecules (capture biomolecules) capable of recognizing IgE selected from the list consisting of the different combinations of peptides defined in the method of the first aspect of the invention or in any of the preferred embodiments thereof; and

b. A support where the recognition biomolecules of step a) are immobilized; and

c. A second recognition molecule (detection biomolecule) capable of recognizing the target analyte or the capture biomolecule optionally bound to a tag molecule.

A fifth aspect of the invention relates to the use of the kit of the fourth aspect of the invention or of any of the preferred embodiments thereof, or the composition of the third aspect of the invention or the peptide microarray of the second aspect of the invention, for prognosis of the number of reactions during oral immunotherapy (OIT) against proteins in cow's milk and/or an estimate of the treatment time required to achieve tolerance or desensitization during OIT and/or the need to administer pre-medication during OIT.

In a sixth aspect of the invention, the present invention relates to a method for prognosis and follow-up of the treatment of allergy to proteins in cow's milk with oral immunotherapy with cow's milk which comprises determining the presence of IgE or IgG4 antibodies against the peptides identified with peptide sequences SEQ ID NO: 1-17 and/or fragments thereof in a sample isolated from a subject.

In a particular embodiment of the sixth aspect of the invention, a peptide recognition of less than 15% (peptides recognized by IgE antibodies in the biological sample of step a) is indicative of a good prognosis.

In a particular embodiment of the sixth aspect of the invention, a peptide recognition comprised between 15%-75% (peptides recognized by IgE antibodies in the biological sample of step a) is indicative of a moderate prognosis.

In a particular embodiment of the sixth aspect of the invention, a peptide recognition of more than 75% (peptides recognized by IgE antibodies in the biological sample of step a) is indicative of a poor prognosis.

In a particular embodiment of the sixth aspect of the invention, the fragments of the peptides (those recognized by IgE antibodies in the biological sample of step a) are identified with sequences SEQ ID NO: 18-75.

In a particular embodiment of the sixth aspect of the invention, the sample to be analyzed is selected from blood, serum or urine.

In a particular embodiment of the sixth aspect of the invention, the presence of peptides and/or fragments thereof is determined by means of an immunoassay.

In a particular embodiment of the sixth aspect of the invention, the presence of peptides and/or fragments thereof is determined by means of peptide microarrays.

A seventh aspect of the invention relates to a microarray for prognosis and follow-up of the treatment of allergy to proteins in cow's milk with oral immunotherapy with cow's milk according to the method of the sixth aspect of the present invention comprising peptides of peptide sequences SEQ ID NO: 1-17 and/or fragments thereof.

A eighth aspect of the invention relates to a kit for prognosis and follow-up of the treatment of allergy to proteins in cow's milk with oral immunotherapy with cow's milk according to the method of the sixth aspect of the present invention, comprising peptides of peptide sequences SEQ ID NO: 1-17 and/or fragments thereof.

In another aspect, the present invention relates to the use of the kit of the eighth aspect of the invention for prognosis and follow-up of the treatment of allergy to proteins in cow's milk with oral immunotherapy with cow's milk.

## BRIEF DESCRIPTION OF THE DRAWINGS

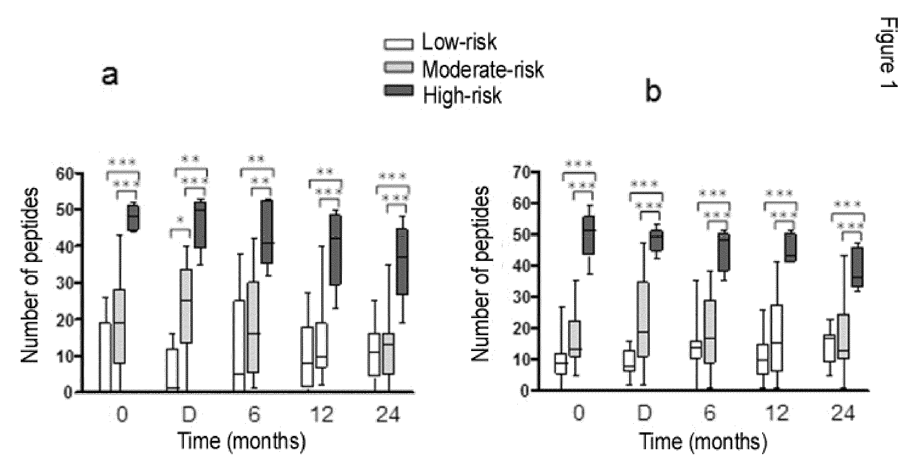

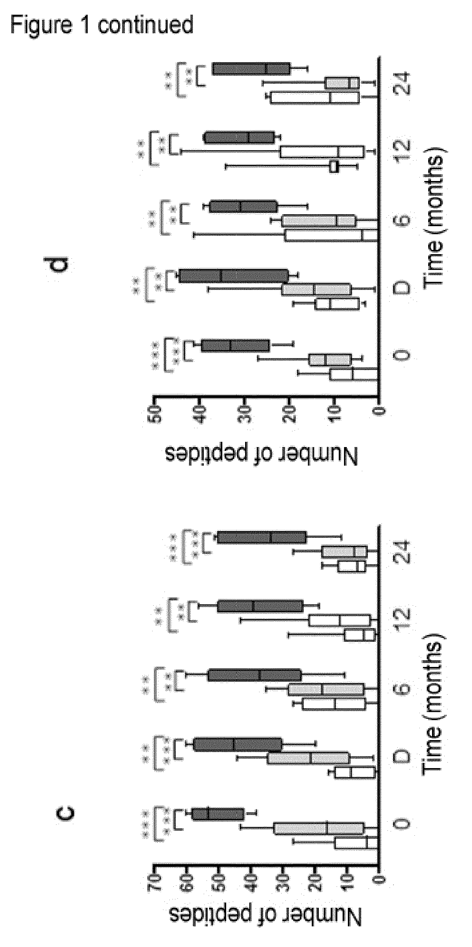

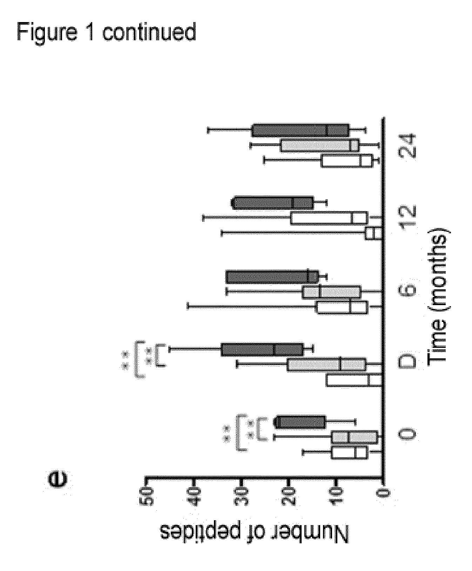

Figure 1 depicts the time course of the number of peptides recognized by patients by means of IgE (0, time before starting OIT treatment; D, time in which tolerance/desensitization is reached; 6, 12 and 24 months of follow-up after desensitization). High-risk patients recognize a greater number of peptides by IgE even before starting OIT treatment, which allows distinguishing high-risk patients from moderate- and low-risk patients. The number of IgE peptides recognized by the different groups of patients decreases with follow-up time which provides information about the minimum duration the OIT must have with cow's milk.
a: IgE-alpha-S1-casein; b: IgE-alpha-S2-casein, c: IgE-b-casein, d: IgE-b-lactoglobulin, e: IgE-k-casein.

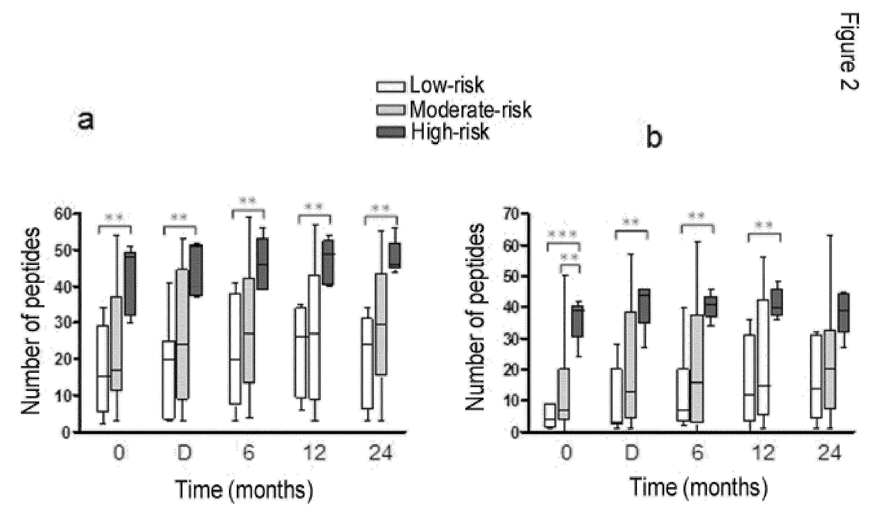

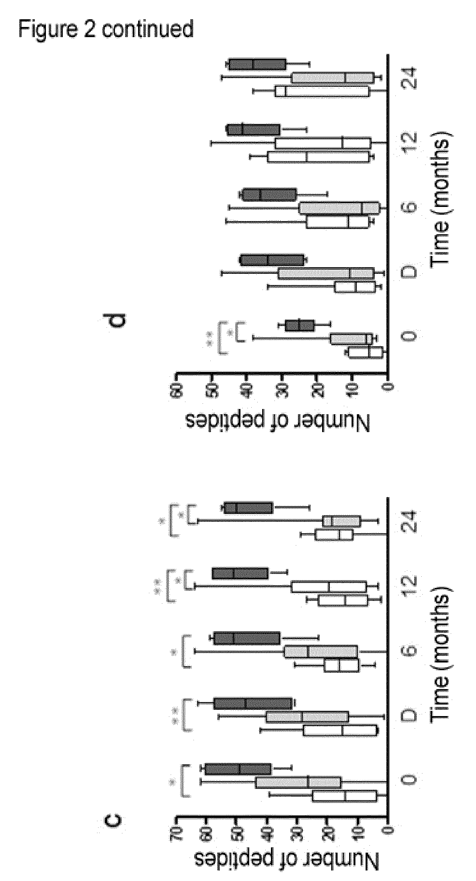

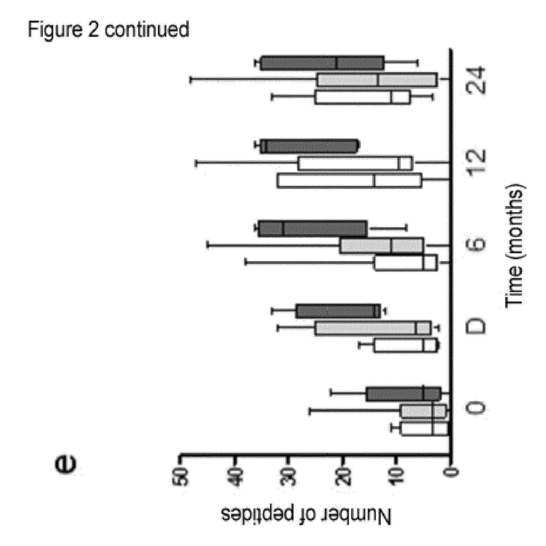

Figure 2 depicts the time course of the number of peptides recognized by patients' IgG4 (0, time before starting OIT treatment; D, time in which tolerance/desensitization is reached; 6, 12 and 24 months of follow-up after desensitization). The number of peptides recognized by the different groups of patients increases during follow-up time which is indicative of the maintenance of tolerance and therefore the efficacy of the OIT treatment with cow's milk.
a: IgG4-alpha-S1-casein; b: IgG4-alpha-S2-casein, c: IgG4-b-casein, d: IgG4-b-lactoglobulin, e: IgG4-k-casein.

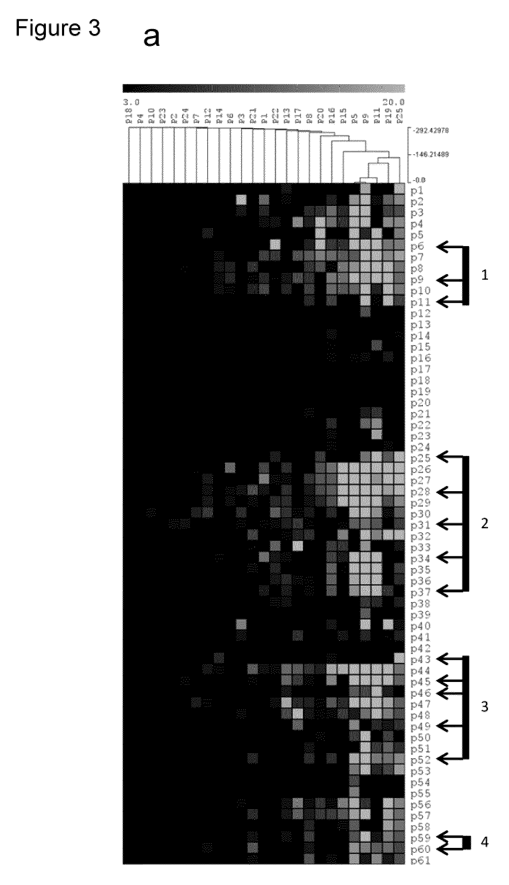

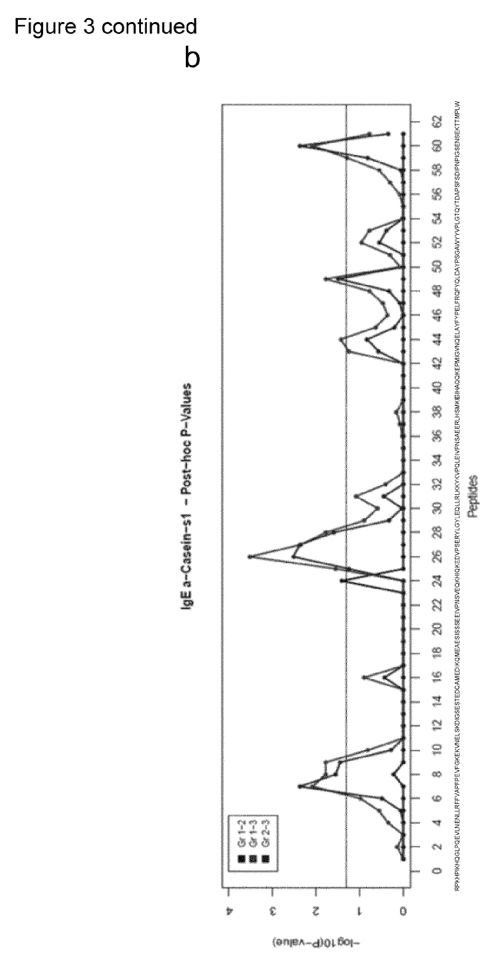

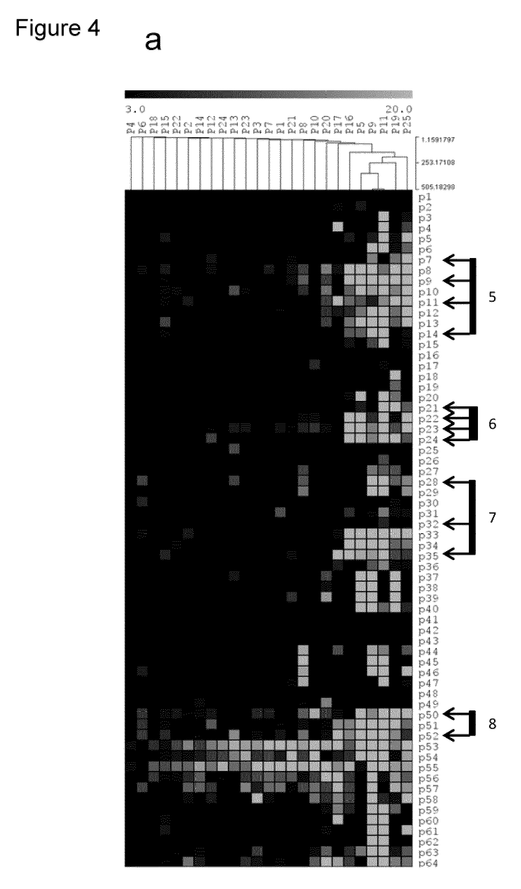

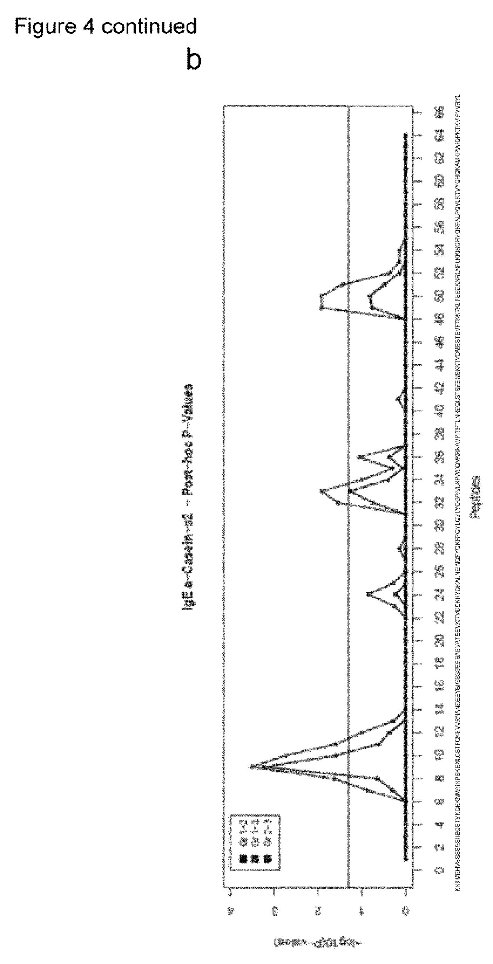

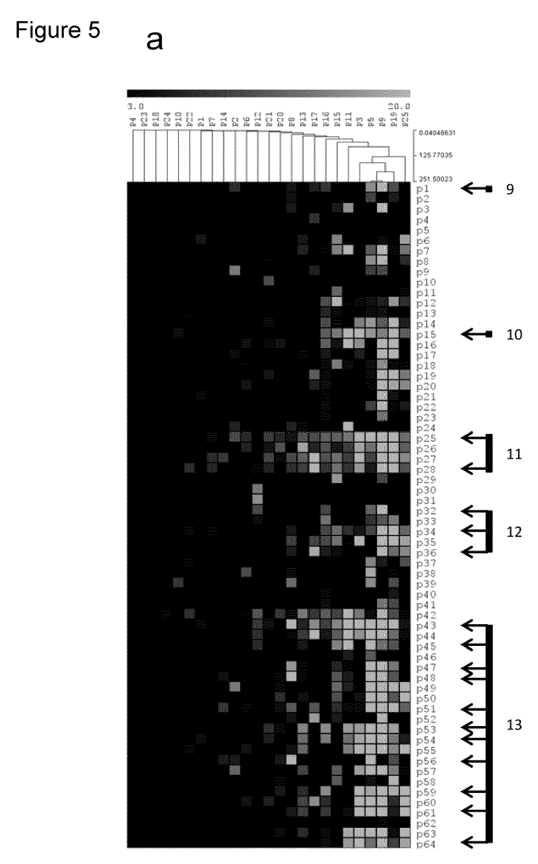

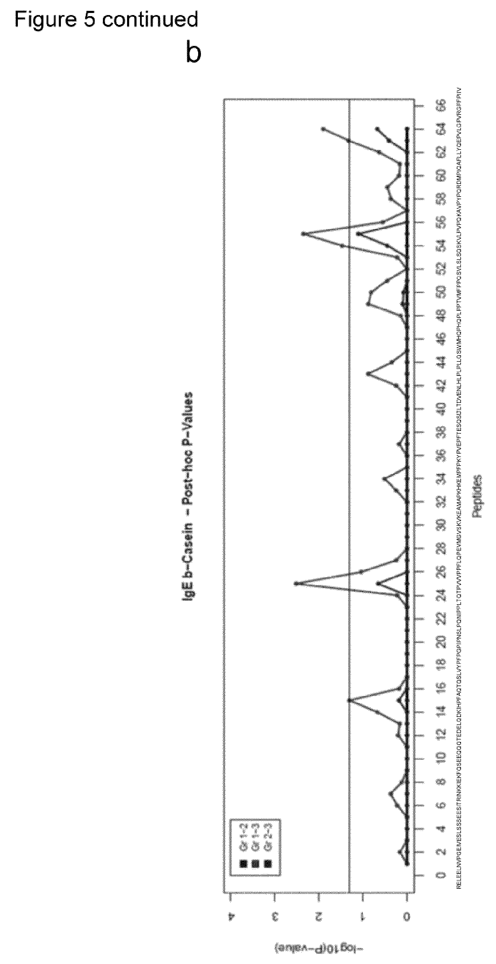

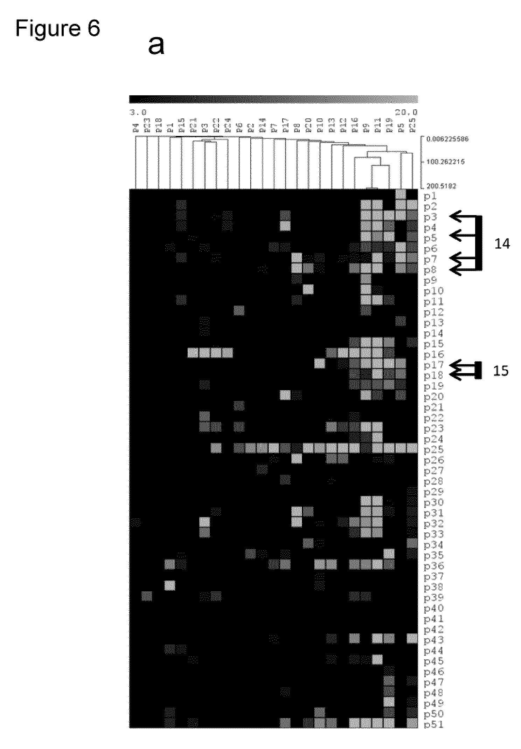

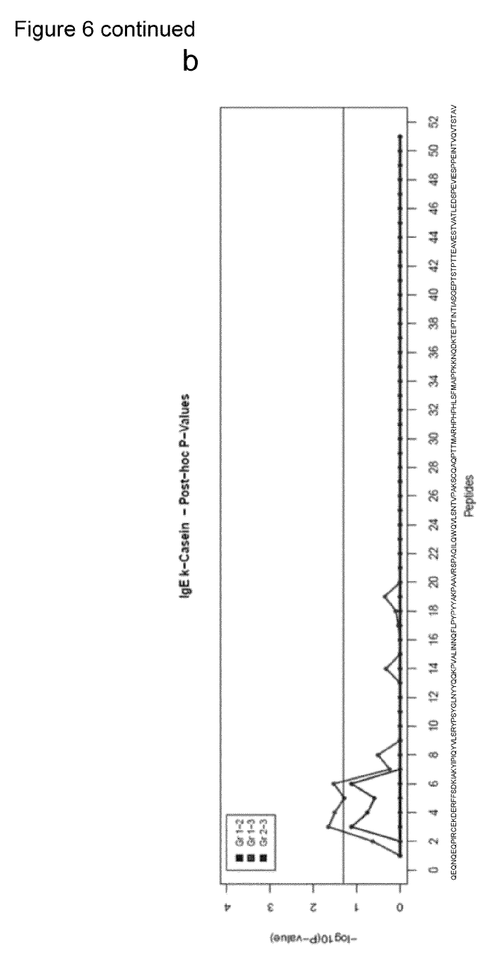

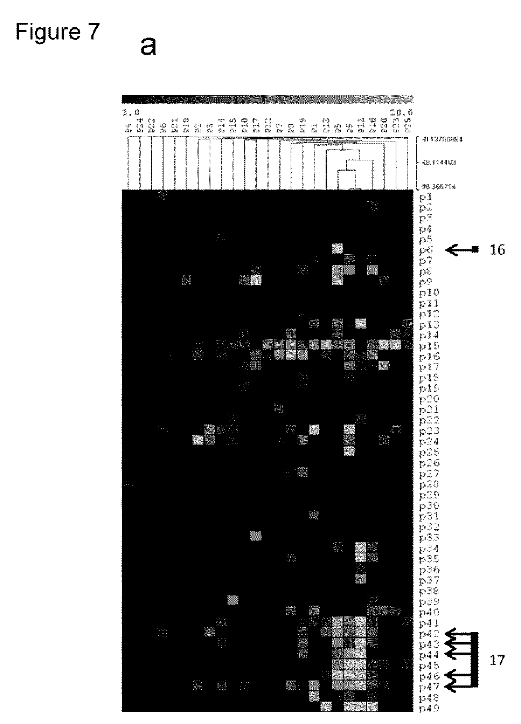

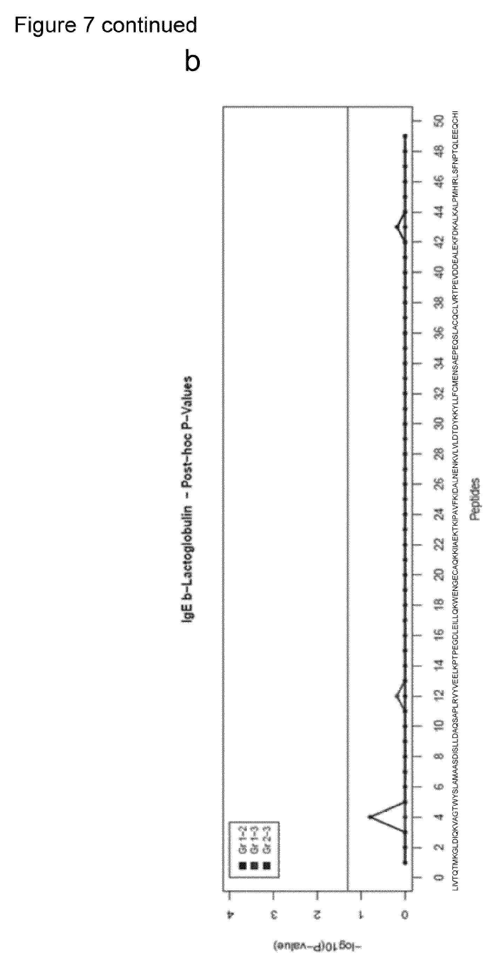

Figures 3 to 7 show the hierarchical cluster analysis, the statistical analysis of the different regions recognized by the different groups of patients (statistical analysis -Gr 1 low-risk patients, Gr 2 moderate-risk patients and Gr 3 high-risk patients-) and the selection of important peptides (key peptide biomarker) (arrows) for patient classification. By means of cluster analysis it can be seen that the immunological recognition patterns by IgE antibodies before starting treatment (time 0) on the high-risk patients are very similar and they are grouped on the righthand side of the graph (p5, p9, p11, p19 and p25). Those zones or epitopes with significant differences in the recognition pattern of high-risk patients with respect to low- and moderate-risk patients have been located by means of statistical analysis. The best peptides for estimating the progression of OIT treatment have been located by means of machine learning bioinformatics analysis. Fifty-eight peptides (see sequences in Figure 8) of alpha-s1-casein, alpha-s2-casein, beta-casein, kappa-casein and beta-lactoglobulin proteins which are important for estimating the progression of OIT treatment have been selected based on the foregoing,

Figure 3: alpha-S1-casein (IgE), unweighted,
Figure 4: alpha-S2-casein (IgE), unweighted,
Figure 5: b-casein (IgE), unweighted,
Figure 6: k- casein (IgE), unweighted,
Figure 7: b-lactoglobulin (IgE), unweighted.

Figure 8 shows the linear correlation between the number of recognized peptides from the 58 peptides shown in Table I and the number of allergic reactions during OIT treatment ($r^2$ 0.803) and the treatment time required to achieve tolerance or desensitization ($r^2$ 0.601) in different patients.

Figure 9 shows the correlation between the number of peptides from that list recognized by IgE before starting OIT treatment and the number of allergic reactions during treatment of different patients

Figure 10 shows the correlation between the number of peptides from the list (Table 3) recognized by IgE before starting OIT treatment and the treatment time required to achieve tolerance or desensitization in different patients.

## DETAILED DISCLOSURE OF THE INVENTION

Definitions

In the context of the present invention, a "good prognosis" refers to a low probability of having adverse reactions during desensitization (<7 reactions) and requiring a short time to achieve tolerance to cow's milk (< 8 weeks) and not requiring pre-medication (both antihistamines and corticoids).

In the context of the present invention, a "moderate prognosis" refers to a high number of allergic reactions during desensitization (>7 reactions) or requiring a long time to achieve tolerance to cow's milk (>8 weeks) or requiring antihistamines as pre-medication.

In the context of the present invention, a "poor prognosis" refers to a high number of allergic reactions during desensitization (>7 reactions) and requiring a long time to achieve tolerance to cow's milk (>8 weeks) and requiring antihistamines and corticoids as pre-medication.

In the context of the present invention, tolerance/desensitization is defined as the consumption of 200 ml of cow's milk without having adverse reactions.

In the context of the present invention, pre-medication refers to the administration of antihistamines and/or corticoids before each of the phases of the OIT.

In the context of the present invention, "IgE antibodies against or with specificity for" refers to IgE antibodies recognizing any of the sequences of the 58 epitopes/peptides shown in Table 1, preferably with an intensity threshold equal to or greater than 3 (z-score>3) read in a ScanArray Express fluorescence scanner (PerkinElmer, Waltham, MA, USA) with a 543 nm laser for detecting the signal of IgE-Alexa 546.

In the context of the present invention, derivatives of sequences of the invention that are immunologically active are understood as those fragments of any of sequences SEQ ID No 19-75 that can be epitopes or antigenic determinants to which type E immunoglobulins (IgE) bind, preferably with an intensity threshold equal to or greater than 3 (z-score>3) read in a ScanArray Express fluorescence scanner (PerkinElmer, Waltham, MA, USA) with a 543 nm laser for detecting the signal of IgE-Alexa 546, of patients allergic to cow's milk and which can be determined by means of an immunoassay.

In the context of the present invention, type E immunoglobulins (IgE) is understood as the isotype of type E immunoglobulins, also known as type E plasma antibodies. IgG4 is understood as the isotype of type G4 immunoglobulins, also known as type G4 plasma antibodies.

**DETAILED DISCLOSURE OF THE INVENTION**

The immunological recognition pattern (IgE and IgG4 recognition) for proteins in cow's milk (alpha-s1-casein, alpha-s2-casein, beta-casein, kappa-casein and beta-lactoglobulin) in allergic patients during OIT treatment was analyzed by means of peptide microarrays. The times analyzed were:

- Before treatment (0)
- Once tolerance is achieved by means of OIT (D)
- 6, 12 and 24 months after achieving tolerance (6, 12 and 24, respectively).

An OIT protocol for the administration of cow's milk in two phases was designed, an initial phase consisting of four days with progressive doses each day and another six days administering a single dose twice a week until reaching the full dose of 200 ml of cow's milk. This guideline was modified according to the tolerance to same, occurrence of intercurrent pathologies not related to the study (fever, cold episodes, other infectious processes particularly digestive processes, etc.). All the reactions that appeared during administration of cow's milk both in the hospital and outside the health facility were recorded and quantified.

Patients with persistent allergy to proteins in cow's milk were selected.

The patient inclusion criteria were:

Children over 4 years of age diagnosed with APCW who would not have achieve tolerance spontaneously.

To check the persistence of APCW during the inclusion of patients into the study, skin testing and determination of IgE specific against cow's milk and fractions thereof (beta-lactoglobulin, alpha-lactalbumin and casein) were carried out and a double-blind, placebo-controlled provocation was performed with respect to cow's milk.

Positive skin testing and the presence of IgE specific against cow's milk or any of its fractions and a positive provocation with exposure to the cow's milk was required to reproduce immediate allergy symptoms.

Skin testing was considered positive when it had a size equal to or greater than that produced by histamine and determination of specific IgE was considered positive when it exceeded 0.35 kU/L determined by CAP (Thermo Fisher Scientific).

Children diagnosed with APCW who failed to comply with the previously defined criteria and children with lactose intolerance or reactions not mediated by IgE were excluded.

A total of 47 patients were included, from that 12 were excluded as they showed an exclusion criterion. A total of 35 children complied with the inclusion criteria and they were all offered OIT treatment with cow's milk. From the 35 children, a total of 24 accepted the treatment and another 7 children rejected the treatment but agreed to remain in the study as a control group.

In terms of the efficacy of the treatment, efficacy was considered reaching the amount of 200 ml of cow's milk a day without adverse reactions.

In terms of the safety of the guideline of OIT with cow's milk, the number of allergic reactions presented by each patient during the procedure, the need for protection with anti-allergy medication (corticoids and antihistamines) and the duration of the guideline were considered.

Twenty-four included patients reached the dose of 200 ml, therefore the procedure was effective in all cases.

The patients were classified according to duration longer or less than 8 weeks (median procedure duration with all patients), number of reactions of more than 7 (median group reactions) and whether or not they need anti-allergy medication (antihistamines and/or corticoids) for administration of the doses (Yes, No).

According to these criteria, the patients were classified into three groups

- Low risk (less than 8 weeks, less than 7 reactions and do not need medication). Seven patients showed this profile,
- Moderate risk (more than 8 weeks and/or more than 7 reactions and/or pre-medication with antihistamines). Twelve patients showed this profile,
- High risk (more than 8 weeks and more than 7 reactions and complete pre-medication (antihistamines and corticoids). Five patients showed this profile.

Once the test was performed, the authors of the present invention correlated the number of peptides recognized by IgE antibodies in the sera of the patients for the different proteins before starting OIT treatment (time 0) with the response after said treatment. The low-risk patients recognized a few peptides, the high-risk patients recognized a lot of peptides and the moderate-risk patients recognized an intermediate number of peptides. Fifty-eight epitopes/peptides of the five proteins responsible for APCW were identified from this test, providing information for prognosis of the response to OIT against cow's milk (Table 1 and Figures 3 to 7). Furthermore, the time course in the recognition of these 58 epitopes/peptides by IgE (and optionally IgG4) allows determining the duration of the OIT (increased IgG4 recognition and decreased IgE recognition), and indicating the patient's immunological tolerance/desensitization status (the patient tolerates food requiring uninterrupted, daily exposure to the allergen) or permanent tolerance (the patients tolerates food and suspension thereof for a time period, the state of tolerance is unchanged), and therefore the overall efficacy of the OIT with cow's milk.

By means of bioinformatics analysis, a subgroup of 27 epitopes/peptides having a high prognosis value for prognosis of the number of reactions during OIT (Table 3 and Figure 9) and the treatment time required to achieve tolerance (Table 4 and Figure 10) was located from among the 58 epitopes/peptides described above.

Table 3 shows reference values based on 16 peptides recognized by IgE antibodies in the sera of 24 patients for estimating the number of reactions during oral immunotherapy (OIT).

Table 4 shows reference values based on 16 peptides recognized by IgE antibodies in the sera of 24 patients for estimating the treatment time required to achieve tolerance or desensitization during OIT.

To correlate the number of peptide sequences recognized by IgE antibodies in the sera of the subjects before starting OIT treatment (time 0) with the reference values established based on those same peptide sequences which correlate the number of peptides recognized by IgE antibodies in the sera of the patients with the response after OIT treatment, algorithms or mathematical formulas, such as for example, regression lines such as those included in Table 3 and 4, can be used.

Therefore, by way of example, the reference values obtained from the 24 patients previously analyzed could be used for estimating the number of reactions during OIT and the treatment time required to achieve tolerance or desensitization. Therefore, for estimating the number of reactions, the relationship between the number of peptides in Table 2 recognized by each of the 24 reference patients and the number of reactions each of them experienced during OIT could be modeled by means of a linear fit or linear regression model (Figure 9), thereby establishing an equation which allows estimating in new patients the number of reactions they will experience during OIT based on the number of recognized peptides before starting treatment.

Additionally and for estimating the treatment time required to achieve tolerance or desensitization during OIT, the relationship between the number of peptides in Table 3 recognized by each of the 24 reference patients and the time required by each of them to achieve tolerance could be modeled by means of a linear fit or linear regression model (Figure 10) and thereby establishing an equation which allows estimating in new patients the time required to achieve tolerance during OIT based on the number of recognized peptides before starting treatment.

Therefore, as can be seen in Figures 8-10, by analyzing the IgE recognition pattern of the immunologically active sequences of the 58 epitopes/peptides shown in Table 1, as well as of subsets of said sequences such as the 16 peptides shown in Table 3 or the 16 peptides shown in Table 4, a series of reference values (see Figures 8-10) have been established which allows, depending on the number of peptides recognized by IgE in a sample isolated from a patient before starting OIT treatment, estimating the number of adverse reactions and the time required to achieve desensitization as well as whether or not pre-medication is needed. Furthermore, it allows classifying the patients as low-risk, moderate-risk or high-risk patients depending on the number of peptides recognized by IgE before starting the OIT.

Table 1

| ID | Protein | Position | Sequence |
|----|---------|----------|----------|
| 1 | alpha-s1-casein | 6 | SEQ ID NO: 18 |
| 2 | alpha-s1-casein | 9 | SEQ ID NO: 19 |
| 3 | alpha-s1-casein | 11 | SEQ ID NO: 20 |
| 4 | alpha-s1-casein | 25 | SEQ ID NO: 21 |
| 5 | alpha-s1-casein | 28 | SEQ ID NO: 22 |
| 6 | alpha-s1-casein | 31 | SEQ ID NO: 23 |
| 7 | alpha-s1-casein | 34 | SEQ ID NO: 24 |
| 8 | alpha-s1-casein | 37 | SEQ ID NO: 25 |
| 9 | alpha-s1-casein | 43 | SEQ ID NO: 26 |
| 10 | alpha-s1-casein | 45 | SEQ ID NO: 27 |
| 11 | alpha-s1-casein | 46 | SEQ ID NO: 28 |
| 12 | alpha-s1-casein | 49 | SEQ ID NO: 29 |

(continued)

| ID | Protein | Position | Sequence |
|----|---------|----------|----------|
| 13 | alpha-s1-casein | 52 | SEQ ID NO: 30 |
| 14 | alpha-s1-casein | 59 | SEQ ID NO: 31 |
| 15 | alpha-s1-casein | 60 | SEQ ID NO: 32 |
| 16 | alpha-s2-casein | 7 | SEQ ID NO: 33 |
| 17 | alpha-s2-casein | 9 | SEQ ID NO: 34 |
| 18 | alpha-s2-casein | 11 | SEQ ID NO: 35 |
| 19 | alpha-s2-casein | 14 | SEQ ID NO: 36 |
| 20 | alpha-s2-casein | 21 | SEQ ID NO: 37 |
| 21 | alpha-s2-casein | 22 | SEQ ID NO: 38 |
| 22 | alpha-s2-casein | 23 | SEQ ID NO: 39 |
| 23 | alpha-s2-casein | 24 | SEQ ID NO: 40 |
| 24 | alpha-s2-casein | 28 | SEO ID NO: 41 |
| 25 | alpha-s2-casein | 32 | SEQ ID NO: 42 |
| 26 | alpha-s2-casein | 35 | SEQ ID NO: 43 |
| 27 | alpha-s2-casein | 50 | SEO ID NO: 44 |
| 28 | alpha-s2-casein | 52 | SEO ID NO: 45 |
| 29 | beta-casein | 1 | SEQ ID NO: 46 |
| 30 | beta-casein | 15 | SEO ID NO: 47 |
| 31 | beta-casein | 25 | SEQ ID NO: 48 |
| 32 | beta-casein | 28 | SEQ ID NO: 49 |
| 33 | beta-casein | 32 | SEQ ID NO: 50 |
| 34 | beta-casein | 34 | SEQ ID NO: 51 |
| 35 | beta-casein | 36 | SEQ ID NO: 52 |
| 36 | beta-casein | 43 | SEQ ID NO: 53 |
| 37 | beta-casein | 45 | SEO ID NO: 54 |
| 38 | beta-casein | 47 | SEQ ID NO: 55 |
| 39 | beta-casein | 48 | SEQ ID NO: 56 |
| 40 | beta-casein | 51 | SEQ ID NO: 57 |
| 41 | beta-casein | 53 | SEQ ID NO: 58 |
| 42 | beta-casein | 54 | SEQ ID NO: 59 |
| 43 | beta-casein | 56 | SEQ ID NO: 60 |
| 44 | beta-casein | 59 | SEQ ID NO: 61 |
| 45 | beta-casein | 61 | SEQ ID NO: 62 |
| 46 | beta-casein | 64 | SEQ ID NO: 63 |
| 47 | kappa-casein | 3 | SEQ ID NO: 64 |
| 48 | kappa-casein | 5 | SEQ ID NO: 65 |
| 49 | kappa-casein | 7 | SEQ ID NO: 66 |
| 50 | kappa-casein | 8 | SEQ ID NO: 67 |

(continued)

| ID | Protein | Position | Sequence |
|----|---------|----------|----------|
| 51 | kappa-casein | 17 | SEQ ID NO: 68 |
| 52 | kappa-casein | 18 | SEQ ID NO: 69 |
| 53 | beta-lactoglobulin | 6 | SEQ ID NO: 70 |
| 54 | beta-lactoglobulin | 42 | SEQ ID NO: 71 |
| 55 | beta-lactoglobulin | 43 | SEQ ID NO: 72 |
| 56 | beta-lactoglobulin | 44 | SEQ ID NO: 73 |
| 57 | beta-lactoglobulin | 46 | SEO ID NO: 74 |
| 58 | beta-lactoglobulin | 47 | SEQ ID NO: 75 |

Table 1 shows the 58 epitopes/peptides of the five proteins responsible for APCW.

Table 2 shows the 17 sequences (see Figures 3-7) included in the 58 epitopes/peptides of the five proteins responsible for APCW.

Table 2

| ID | Sequence |
|----|----------|
| 1 | LNENLLRFFVAPFPEVFGKEKVNELSKDIGSESTE |
| 2 | PNSVEQKHIQKEDVPSERYLGYLEQLLRLKKYKVPQLEIVPNSAEERLHSMKEGIH |
| 3 | IHAQQKEPMIGVNQELAYFYPELFRQFYQLDAYPSGAWYYVPLGTQ |
| 4 | DAPSFSDIPNPIGSENSEKTTMP |
| 5 | TYKQEKNMAINPSKENLCSTFCKEWRNANEEEYSIGSSSE |
| 6 | SAEVATEEVKITVDDKHYQKALNEINQFY |
| 7 | LNEINQFYQKFPQYLQYLYQGPIVLNPWDQVKRNAVPITPT |
| 8 | TKKTKLTEEEKNRLNFLKKISQRYQK |
| 9 | RELEELNVPGEIVESLSSSE |
| 10 | DELQDKIHPFAQTQSLVYPF |
| 11 | NIPPLTQTPVVVPPFLQPEVMGVSKVKEA |
| 12 | GVSKVKEAMAPKHKEMPFPKYPVEPFTESQSL |
| 13 | LTDVENLHLPLPLLQSWMHQPHQPLPPTVMFPPQSVLSLSQSKVLPVPQKAVPYPQRDMPIQAFLLYQEPVLGPVRGPFPIIV |
| 14 | QPIRCEKDERFFSDKIAKYIPIQYVLSRYPSYGLN |
| 15 | ALINNQFLPYPYYAKPAAVRSPA |
| 16 | AGTWYSLAMAASDISLLDAQ |
| 17 | RTPEVDDEALEKFDKALKALPMHIRLSFNPTQLEE |

The following examples are merely intended for illustrating the invention and in no case for limiting same.

Example 1:

Using a SpotArray 72 microarrays printing robot (PerkinElmer, Waltham, MA, USA) microarrays were printed on NSB27 NHS glass functionalized with N-hydroxyl succinimidyl (NanoSurface Biosciences-POSTECH, Seoul, Korea) containing 27 peptides with ID: 2 ($\alpha$-s1-casein p9), 4 ($\alpha$-s1-casein p25), 8 ($\alpha$-s1-casein p37), 10 ($\alpha$-s1-casein p45), 11 ($\alpha$-s1-casein p46), 14 ($\alpha$-s1-casein p59), 19 (alpha-s2-casein p14), 20 (alpha-s2-casein p21), 21 (alpha-s2-casein p22), 22 (alpha-s2-casein p23), 23 (alpha-s2-casein p24), 27 (alpha-s2-casein p50), 34 (beta-casein p34), 36 (beta-casein p43), 38 (beta-casein p47), 39 (beta-casein p48), 41 (beta-casein p53), 42 (beta-casein p54), 45 (beta-casein p61), 50 (kappa-casein p8), 51 (kappa-casein p17), 52 (kappa-casein p18), 53 (beta-lactoglobulin p6), 54 (beta-lactoglobulin p42), 55 (beta-lactoglobulin p43), 56 (beta-lactoglobulin p44) and 58 (beta-lactoglobulin p47); see Table 3 and 4 and Figure 9 and 10. For printing, the peptides are dissolved in a 1:1 phosphate saline buffer (PBS) solution and printing buffer (PPB) (protein printing buffer, Arrayit Corporation, Sunnyvale, CA, USA). Once the microarrays were printed, non-specific bindings were blocked for 1 hour at room temperature with a 1:1 BlockIt™ blocking solution (Arrayit Corporation, Sunnyvale, CA, USA) and PBS containing 0.1% Tween 20 and 2% Bovine Serum Albumin (PBS-T-BSA).

The sera obtained from 3 patients were incubated on the surface of the microarrays for 16 hours at 4°C and under continuous stirring. The sample was removed from the microarrays and washed twice with a PBS-T-BSA solution. They were then incubated with a solution containing anti-human IgE fluorescent antibodies (G7-26, BD-PharMingen, San Diego, CA, USA) labeled with Alexa 546 and anti-human IgG4 fluorescent antibodies (G14-4, BD-PharMingen, San Diego, CA, USA) labeled with Alexa 647, diluted at 1/1000 in PBS-T-BSA. The solution containing antibodies was removed and washed twice with a PBS-T-BSA solution.

To detect the presence in the serum of the patients of IgE and IgG4 antibodies against the different peptides of the allergen proteins in cow's milk, the microarrays were read in a ScanArray Express fluorescence scanner (PerkinElmer, Waltham, MA, USA) with two lasers (a 543 nm laser for detecting the signal of IgE-Alexa 546 and a 633 nm laser for detecting the signal of IgG4-Alexa 647). The results were quantified and standardized for transforming the fluorescence signal into a numerical parameter (arbitrary fluorescence units or z-score). The number of peptides with a fluorescence signal greater than 3 (z-score>3) was calculated. The first patient recognized 2 peptides, the second patient recognized 8 peptides and the third patient recognized 15 peptides.

The patient with a peptide recognition of 2 has a good prognosis (low probability of having adverse reactions during desensitization - an estimate of 3 allergic reactions, see Table 3 and Figure 9- and requiring a short time to achieve tolerance to cow's milk - an estimate of 7 weeks, see Table 4 and Figure 10-).

Patients with a peptide recognition of 8 has an intermediate prognosis (moderate risk of having adverse reactions during desensitization - an estimate of 10 allergic reactions, see Table 3 and Figure 9- and requiring an intermediate time to achieve tolerance to cow's milk - an estimate of 11 weeks, see Table 4 and Figure 10-).

Patients with a peptide recognition of 15 has a poor prognosis (high probability of having adverse reactions during desensitization - an estimate 19 allergic reactions, see Table 2 and Figure 9- and requiring a long time to achieve tolerance to cow's milk - an estimate of 16 weeks. Additionally, these patients will require pre-medication and treatment with antihistamines and corticoids before each of the phases of the OIT (see Table 4 and Figure 10).

Table 3

| ID | Protein | Sequence |
|----|---------|----------|
| 2 | $\alpha$-s1-casein p9 | SEQ ID NO:19 |
| 4 | $\alpha$-s1-casein p25 | SEQ ID NO: 21 |
| 8 | $\alpha$-s1-casein p37 | SEQ ID NO: 25 |
| 10 | $\alpha$-s1-casein p45 | SEQ ID NO: 27 |
| 19 | alpha-s2-casein p14 | SEQ ID NO: 36 |
| 20 | alpha-s2-casein p21 | SEQ ID NO: 37 |
| 21 | alpha-s2-casein p22 | SEQ ID NO: 38 |
| 34 | beta-casein p34 | SEQ ID NO: 51 |
| 36 | beta-casein p43 | SEQ ID NO: 53 |
| 41 | beta-casein p53 | SEQ ID NO: 58 |
| 42 | beta-casein p54 | SEQ ID NO: 59 |
| 45 | beta-casein p61 | SEQ ID NO: 62 |
| 52 | kappa-casein p18 | SEQ ID NO: 69 |
| 54 | beta-lactoglobulin p42 | SEO ID NO: 71 |
| 55 | beta-lactoglobulin p43 | SEQ ID NO: 72 |
| 56 | beta-lactoglobulin p44 | SEQ ID NO: 73 |

Table 3: peptides that best estimate the number of reactions during OIT Formula: Estimated reactions =1.1733 No. of peptides + 1.0362

No. of peptides: 2, Estimated reactions: 3
No. of peptides: 8, Estimated reactions: 10
No. of peptides: 15, Estimated reactions: 19

Table 4

| ID | Peptide | Sequence |
|---|---|---|
| 11 | α-s1-casein p46 | SEQ ID NO: 28 |
| 14 | α-s1-casein p59 | SEQ ID NO: 31 |
| 19 | alpha-s2-casein p14 | SEQ ID NO: 36 |
| 20 | alpha-s2-casein p21 | SEQ ID NO: 37 |
| 21 | alpha-s2-casein p22 | SEQ ID NO: 38 |
| 22 | alpha-s2-casein p23 | SEQ ID NO: 39 |
| 23 | alpha-s2-casein p24 | SEQ ID NO: 40 |
| 27 | alpha-s2-casein p50 | SEO ID NO: 44 |
| 36 | beta-casein p43 | SEQ ID NO: 53 |
| 38 | beta-casein p47 | SEQ ID NO: 55 |
| 39 | beta-casein p48 | SEQ ID NO: 56 |
| 50 | kappa-casein p8 | SEQ ID NO: 67 |
| 51 | kappa-casein p17 | SEQ ID NO: 68 |
| 53 | beta-lactoglobulin p6 | SEQ ID NO: 70 |
| 55 | beta-lactoglobulin p43 | SEQ ID NO: 72 |
| 58 | beta-lactoglobulin p47 | SEQ ID NO: 75 |

Table 4: peptides that best estimate the treatment time required to achieve tolerance or desensitization during OIT.

Formula: Estimated treatment time (weeks) = (0.7318 * No. of peptides) + 5.1419

No. of peptides: 2, Estimated treatment time (weeks): 7
No. peptides: 8, Estimated treatment time (weeks): 11
No. peptides: 15, Estimated treatment time (weeks): 16

**Claims**

1. A method for prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT in a human subject, comprising the following steps:

c. Isolating a biological sample from the subject;

d. Determining the presence or absence of IgE antibodies in the biological sample of step a) against or with specificity for peptides identified with the following sequences:

    a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and/or

    b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and/or

    c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and/or

    d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and/or

    e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active;

where the prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment.

2. The method according to claim 1, where the determination of the presence or absence of IgE antibodies of step b) in the biological sample of step a) is performed by determining the presence or absence of IgE antibodies against or with specificity for at least the following peptides identified with the following sequences:

    a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and

    b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and

    c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and optionally

    d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and optionally

    e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active,

3. The method according to claim 1, where the determination of the presence or absence of IgE antibodies of step b) in the biological sample of step a) is performed by determining the presence or absence of IgE antibodies against or with specificity for at least the following peptides identified with the following sequences:

    a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and

    b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and

    c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and

d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and

e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active,

4. The method according to claim 1, where said method is a method for prognosis of the number of reactions during oral immunotherapy (OIT) and the determination of the presence or absence of IgE antibodies of step b) in the biological sample of step a) is performed by determining the presence or absence of IgE antibodies against or with specificity for at least the following peptides identified with the following sequences:

a. sequences SEQ ID NO: 19, 21, 25 and 27 and/or derivatives of sequences SEQ ID NO: 19, 21, 25 and 27 that are immunologically active; and

b. sequences SEQ ID NO: 36, 37 and 38 and/or derivatives of sequences SEQ ID NO: 36, 37 and 38 that are immunologically active; and

c. sequences SEQ ID NO: 51, 53, 58, 59 and 62 and/or derivatives of sequences SEQ ID NO: 51, 53, 58, 59 and 62 that are immunologically active; and

d. sequence SEQ ID NO: 69 and/or a derivative of the sequence SEQ ID NO: 69 that is immunologically active; and

e. sequences SEQ ID NO: 71 and 72 and/or derivatives of sequences SEQ ID NO: 71 and 72 that are immunologically active,

5. The method according to claim 1, where the method is a method for prognosis of an estimate of the treatment time required to achieve tolerance or desensitization during OIT and the determination of the presence or absence of IgE antibodies of step b) in the biological sample of step a) is performed by determining the presence or absence of IgE antibodies against or with specificity for at least the following peptides identified with the following sequences:

a. sequences SEQ ID NO: 28 and 31 and/or derivatives of sequences SEQ ID NO: 28 and 31 that are immunologically active; and

b. sequences SEQ ID NO: 36-40 and 44 and/or derivatives of sequences SEQ ID NO: 36-40 and 44 that are immunologically active; and

c. sequences SEQ ID NO: 53, 55 and 56 and/or derivatives of sequences SEQ ID NO: 53, 55 and 56 that are immunologically active; and

d. sequences SEQ ID NO: 67 and 68 and/or a derivative of the sequence SEQ ID NO: 67 and 68 that are immunologically active; and

e. sequences SEQ ID NO: 70, 72 and 75 and/or derivatives of sequences SEQ ID NO: 70, 72 and 75 that are immunologically active.

6. The method according to claim 1, where the determination of the presence or absence of IgE antibodies in the biological sample of step a) is performed by determining the presence or absence of IgE antibodies against or with specificity for at least the following peptides identified with the following sequences: SEQ ID NO: 18-75 and/or derivatives of sequences SEQ ID NO: 18-75 that are immunologically active.

7. The method according to claim 1, where the determination of the presence or absence of IgE antibodies in the biological sample of step a) is performed by determining the presence or absence of IgE antibodies against or with specificity for at least the following peptides identified with the following sequences: SEQ ID NO: 18-75 and/or derivatives of sequences SEQ ID NO: 18-75 that are immunologically active, where:

- a peptide recognition of less than 15% is indicative of a good prognosis,
- a peptide recognition of between 15%-75% is indicative of a moderate prognosis, and
- a peptide recognition of more than 75% is indicative of a poor prognosis.

8. The method according to claim 1, where the determination of the presence or absence of IgE antibodies in the biological sample of step a) is performed by determining the presence or absence of IgE antibodies against or with specificity for at least the following peptides identified with the following sequences: SEQ ID NO: 18-75 and where:

- a peptide recognition of less than 15% is indicative of a good prognosis,
- a peptide recognition of between 15%-75% is indicative of a moderate prognosis, and
- a peptide recognition of more than 75% is indicative of a poor prognosis.

9. The method according to any of the preceding claims, where the biological sample is selected from biological fluids, such as blood, serum, plasma, cerebrospinal fluid, peritoneal fluid, feces or urine.

10. The method according to any of the preceding claims, where peptide recognition is determined by means of an immunoassay.

11. The method according to any of claims 1-8, where peptide recognition is determined by means of peptide microarrays.

12. A peptide microarray suitable for carrying out the method of any of claims 1-8 comprising the combinations of peptides defined in any of claims 1-8.

13. A composition suitable for carrying out the method of any of claims 1-8, comprising the combinations of peptides defined in any of claims 1-8.

14. A kit suitable for carrying out the method of any of claims 1-8, comprising the combinations of peptides defined in any of claims 1-8.

15. A kit comprising:

a. Recognition molecules (capture biomolecules) capable of recognizing IgE selected from the list consisting of the different combinations of peptides defined in any of claims 1-8; and
b. A second recognition molecule (detection biomolecule) capable of recognizing the target analyte or the capture biomolecule optionally bound to a tag molecule.

16. A kit comprising:

a. Recognition molecules (capture biomolecules) capable of recognizing the IgE selected from the list consisting of the different combinations of peptides defined in any of claims 1-8;
b. A support where the recognition biomolecules of step a) are immobilized; and
c. A second recognition molecule (detection biomolecule) capable of recognizing the target analyte or the capture biomolecule optionally bound to a tag molecule.

17. Use of the kit according to any of claims 14-16 for prognosis of the number of reactions during oral immunotherapy (OIT) against proteins in cow's milk and/or an estimate of the treatment time required to achieve tolerance or desensitization during OIT and/or the need to apply pre-medication during OIT.

Figure 1

Figure 1 continued

Figure 1 continued

Figure 2

Figure 2 continued

Figure 2 continued

e

Figure 3 a

Figure 3 continued

b

IgE a-Casein-s1 – Post-hoc P-Values

## Figure 4

a

# Figure 4 continued

b

Figure 5   a

Figure 5 continued

b

Figure 6

a

## Figure 6 continued

### b

IgE k-Casein – Post-hoc P-Values

Figure 7    a

# Figure 7 continued

## b

Figure 8

a

Figure 8 continued

Figure 9

Figure 10

# INTERNATIONAL SEARCH REPORT

| International application No. |
|---|
| PCT/ES2014/070634 |

### A. CLASSIFICATION OF SUBJECT MATTER

*G01N33/68* (2006.01)

According to International Patent Classification (IPC) or to both national classification and IPC

### B. FIELDS SEARCHED

Minimum documentation searched (classification system followed by classification symbols)

G01N

Documentation searched other than minimum documentation to the extent that such documents are included in the fields searched

Electronic data base consulted during the international search (name of data base and, where practicable, search terms used)

EPODOC, INVENES, WPI, BIOSIS, EMBASE/ELSEVIER, MEDLINE/NLM, XPESP, EMBL-EBI y bases de datos de texto completo TXT

### C. DOCUMENTS CONSIDERED TO BE RELEVANT

| Category* | Citation of document, with indication, where appropriate, of the relevant passages | Relevant to claim No. |
|---|---|---|
| X | WANG J et al. Correlation of IgE/IgG4 milk epitopes and affinity of milk-specific IgE antibodies with different phenotypes of clinical milk allergy.Journal of Allergy and Clinical Immunology, 20100301 ELSEVIER, AMSTERDAM, NL 01/03/2010 VOL: 125 No: 3 Pags: 695 - 702.e6 ISSN 0091-6749 Doi: doi:10.1016/j.jaci.2009.12.017; the whole document. | 4-17 |
| X | CERECEDO INMACULADA "Aplicación from tecnología microarray al manejo diagnóstico from alergia a proteínas from leche of vaca" Tesis Doctoral (May 2009) [retrieved on 23-09-2014]; Retrieved from Internet: URL:<"http://dspace.uah.es/dspace/bitstream/handle/10017/6422/ Versi%c3%b3n%20final%20200905024.pdf?sequence=1">; the whole document. | 4-17 |

☒ Further documents are listed in the continuation of Box C.　　☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 01/10/2014 | **(09/10/2014)** |
| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> M. García Coca <br><br><br> Telephone No. 91 3493411 |

Form PCT/ISA/210 (second sheet) (July 2009)

## INTERNATIONAL SEARCH REPORT

| International application No. |
| --- |
| PCT/ES2014/070634 |

| C (continuation). | DOCUMENTS CONSIDERED TO BE RELEVANT | |
| --- | --- | --- |
| Category * | Citation of documents, with indication, where appropriate, of the relevant passages | Relevant to claim No. |
| X | CERECEDO I et al. "Mapping of the IgE and IgG4 sequential epitopes of milk allergens with a peptide microarray-based immunoassay" Journal of Allergy and Clinical Immunology, 20080901 ELSEVIER, AMSTERDAM, NL 01/09/2008 VOL: 122 No: 3 Pags: 589 - 594 ISSN 0091-6749 Doi: doi:10.1016/j.jaci.2008.06.040; the whole document. | 4-17 |
| X | WO 2011032097 A1 (SINAI SCHOOL MEDICINE ET AL.) 17/03/2011, the whole document. | 4-17 |
| A | SAVILAHTI E M et al. "Early recovery from cow&apos;s milk allergy is associated with decreasing IgE and increasing IgG4 binding to cow&apos;s milk epitopes" Journal of Allergy and Clinical Immunology, 20100601 ELSEVIER, AMSTERDAM, NL 01/06/2010 VOL: 125 No: 6 Pags: 1315 - 1321.e9 ISSN 0091-6749 Doi: doi:10.1016/j.jaci.2010.03.025; the whole document. | 4-17 |
| A | MATSUMOTO N et al. "Peptide array-based analysis of the specific IgE and IgG4 in cow&apos;s milk allergens and its use in allergy evaluation" Peptides, 20091001 ELSEVIER, AMSTERDAM, NL 01/10/2009 VOL: 30 No: 10 Pags: 1840 - 1847 ISSN 0196-9781 Doi: doi:10.1016/j.peptides.2009.07.005 Nachman Ronald J; the whole document. | 4-17 |

Form PCT/ISA/210 (continuation of second sheet) (July 2009)

INTERNATIONAL SEARCH REPORT

| International application No. |
| --- |
| ES2014/070634 |

Continuation of Box II, point 1.

The first step of the claimed method consists in "extracting a biological sample from the patient". This step involves an invasive interaction with the human body, and for this reason this Office considers that the claimed method is a surgical method, since it comprises a step that involves a physical intervention on the human or animal body, which is per se a surgical method. There might be cases in which this step can be eliminated or omitted, whenever said step is not an essential part of the claimed method. This is the case, for example, when a step that consists in a surgical method is a preliminary step carried out before the method according to the invention. This could be the case when a step for extracting samples from the patient is required but this step is not part of the diagnostic method itself performed on the sample.

Continuation of Box II, point 2.

The description (page 18, line 23 - page 19, line 4) indicates that, on the basis of an assay carried out by the authors of the invention, "58 epitopes/peptides of the five proteins involved in cow's milk protein allergy (CMPA) were found which are useful for the prognosis of the response to oral immunotherapy for cow's milk allergy (table 1 and figures 3-7). Moreover, the evolution in time of IgE recognition (and optionally IgG4 recognition) of these 58 epitopes/peptides makes it possible to determine the duration of oral immunotherapy (increase in IgG4 recognition and decrease of IgE recognition), and to indicate the immunological state of the patient, which can be a state of tolerance/desensitisation (the patient tolerates the food substance, requiring daily and uninterrupted exposure to the allergen) or a state of permanent tolerance (the patient tolerates the food substance, and abstention from the food substance for a period of time does not change the state of tolerance), and hence the global effectiveness of oral immunotherapy with cow's milk. Bioinformatics studies located a subgroup of 27 epitopes/peptides among the 58 above-mentioned epitopes/peptides which exhibit a high prognostic value for the number of

reactions during oral immunotherapy (table 3 and figure 9) and for the duration of treatment required to achieve tolerance (table 4 and figure 10)".

Moreover, on page 20, lines 11-21, it is indicated that "through the analysis of the IgE recognition pattern of the immunologically active sequences of the 58 epitopes/peptides shown in table 1, and of subsets of said sequences, such as the 16 peptides shown in table 3 or the 16 peptides shown in table 4, a series of reference values (see figures 8-10) has been determined which, according to the number of IgE-recognised peptides in a sample extracted from a patient before the start of oral immunotherapy, allows the number of adverse reactions and the time required to achieve desensitisation to be estimated, as well as the need or not for pre-medication. Moreover, it allows patients to be classified as low-risk, moderate risk or high-risk patients, according to the number of IgE-recognised peptides, before oral immunotherapy is started".

It is concluded from these indications in the description that the method consists in determining the effectiveness of oral immunotherapy with cow's milk by analysing the IgE recognition pattern of ALL 58 epitopes/peptides, or at least of 27 of these epitopes/peptides, grouped into two groups of 16 epitopes/peptides.

In order to establish the present international search report, the search was carried out in respect of claims 4-11 (in full) and claims 12-17 (in part).

Form PCT/ISA/210 (extra sheet) (July 2009)

## INTERNATIONAL SEARCH REPORT

| International application No. |
| --- |
| ES2014/070634 |

**Box No. I    Nucleotide and/or amino acid sequence(s) (Continuation of item 1.c of the first sheet)**

1. With regard to any nucleotide and/or amino acid sequence disclosed in the international application, the international search was carried out on the basis of a sequence listing filed or furnished:

   a.  (means)

   ☐    on paper

   ☒    in electronic form

   b.  (time)

   ☐    in the international application as filed

   ☐    together with the international application in electronic form

   ☐    subsequently to this Authority for the purposes of search

2. ☐    In addition, in the case that more than one version or copy of a sequence listing has been filed or furnished, the required statements that the information in the subsequent or additional copies is identical to that in the application as filed or does not go beyond the application as filed, as appropriate, were furnished.

3. Additional comments:

   A sequence listing was available to the International Searching Authority (ISA/ES) at the time the international search was carried out, in an acceptable format, and this sequence listing was used for carrying out the international search.

Form PCT/ISA/210 (continuation of first sheet (1)) (July 2009)

## INTERNATIONAL SEARCH REPORT

| International application No. |
|---|
| ES2014/070634 |

**Box No. II**     **Observations where certain claims were found unsearchable (Continuation of item 2 of first sheet)**

This international search report has not been established in respect of certain claims under Article 17(2)(a) for the following reasons:

1. ☐ Claims Nos.: 11.1
   because they relate to subject matter not required to be searched by this Authority, namely:

   Claims 1-11 relate to subject matter which this Authority considers falls under the provisions of PCT Rule 67.1(iv) relating to methods for the treatment of the human or animal body by surgery or therapy, and to diagnostic means. Nevertheless, a search in respect of claims 4-11 has been carried out on the basis of the use of the claimed peptides (see Supplemental Box, point 1).

2. ☐ Claims Nos.: 1-3
   because they relate to parts of the international application that do not comply with the prescribed requirements to such an extent that no meaningful international search can be carried out, specifically:

   Claims 1-3 are not properly supported by the description (see Supplemental Box, point 2).

3. ☐ Claims Nos.:
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## INTERNATIONAL SEARCH REPORT

Information on patent family members

| International application No. |
| --- |
| PCT/ES2014/070634 |

| Patent document cited in the search report | Publication date | Patent family member(s) | Publication date |
| --- | --- | --- | --- |
| WO2011032097 A1 | 17.03.2011 | US2011071043 A1<br>US8802375 B2<br>EP2478373 A1 | 24.03.2011<br>12.08.2014<br>25.07.2012 |

Form PCT/ISA/210 (patent family annex) (July 2009)

## SEQUENCE LISTING

<110> FUNDACIÓN PARA LA INVESTIGACIÓN BIOMÉDICA DEL HOSPITAL
UNIVERSITARIO RAMÓN Y CAJAL
FUNDACIÓN PARA LA INVESTIGACIÓN BIOMÉDICA DEL HOSPITAL
CLINICO SAN CARLOS

<120> MÉTODO PARA EL PRONÓSTICO DE LA EFICACIA DE LA INMUNOTERAPIA ORAL
PARA EL TRATAMIENTO DE LA ALERGIA A LAS PROTEÍNAS DE LECHE DE
VACA

<130> PT0042/2013

<160> 75

<170> PatentIn version 3.5

<210> 1
<211> 35
<212> PRT
<213> Bos taurus

<400> 1

Leu Asn Glu Asn Leu Leu Arg Phe Phe Val Ala Pro Phe Pro Glu Val
1               5                   10                  15


Phe Gly Lys Glu Lys Val Asn Glu Leu Ser Lys Asp Ile Gly Ser Glu
            20                  25                  30


Ser Thr Glu
        35


<210> 2
<211> 56
<212> PRT
<213> Bos taurus

<400> 2

Pro Asn Ser Val Glu Gln Lys His Ile Gln Lys Glu Asp Val Pro Ser
1               5                   10                  15


Glu Arg Tyr Leu Gly Tyr Leu Glu Gln Leu Leu Arg Leu Lys Lys Tyr
            20                  25                  30


Lys Val Pro Gln Leu Glu Ile Val Pro Asn Ser Ala Glu Glu Arg Leu
        35                  40                  45


His Ser Met Lys Glu Gly Ile His
        50                  55


<210> 3
<211> 46
<212> PRT
<213> Bos taurus

<400>   3

```
Ile His Ala Gln Gln Lys Glu Pro Met Ile Gly Val Asn Gln Glu Leu
1               5               10              15


Ala Tyr Phe Tyr Pro Glu Leu Phe Arg Gln Phe Tyr Gln Leu Asp Ala
            20              25              30


Tyr Pro Ser Gly Ala Trp Tyr Tyr Val Pro Leu Gly Thr Gln
        35              40              45
```

<210>   4
<211>   23
<212>   PRT
<213>   Bos taurus

<400>   4

```
Asp Ala Pro Ser Phe Ser Asp Ile Pro Asn Pro Ile Gly Ser Glu Asn
1               5               10              15


Ser Glu Lys Thr Thr Met Pro
            20
```

<210>   5
<211>   41
<212>   PRT
<213>   Bos taurus

<400>   5

```
Thr Tyr Lys Gln Glu Lys Asn Met Ala Ile Asn Pro Ser Lys Glu Asn
1               5               10              15


Leu Cys Ser Thr Phe Cys Lys Glu Val Val Arg Asn Ala Asn Glu Glu
            20              25              30


Glu Tyr Ser Ile Gly Ser Ser Ser Glu
        35              40
```

<210>   6
<211>   29
<212>   PRT
<213>   Bos taurus

<400>   6

```
Ser Ala Glu Val Ala Thr Glu Glu Val Lys Ile Thr Val Asp Asp Lys
1               5               10              15


His Tyr Gln Lys Ala Leu Asn Glu Ile Asn Gln Phe Tyr
            20              25
```

<210> 7
<211> 41
<212> PRT
<213> Bos taurus

<400> 7

Leu Asn Glu Ile Asn Gln Phe Tyr Gln Lys Phe Pro Gln Tyr Leu Gln
1               5                   10                  15

Tyr Leu Tyr Gln Gly Pro Ile Val Leu Asn Pro Trp Asp Gln Val Lys
            20                  25                  30

Arg Asn Ala Val Pro Ile Thr Pro Thr
            35                  40


<210> 8
<211> 26
<212> PRT
<213> Bos taurus

<400> 8

Thr Lys Lys Thr Lys Leu Thr Glu Glu Glu Lys Asn Arg Leu Asn Phe
1               5                   10                  15

Leu Lys Lys Ile Ser Gln Arg Tyr Gln Lys
            20                  25


<210> 9
<211> 20
<212> PRT
<213> Bos taurus

<400> 9

Arg Glu Leu Glu Glu Leu Asn Val Pro Gly Glu Ile Val Glu Ser Leu
1               5                   10                  15

Ser Ser Ser Glu
            20


<210> 10
<211> 20
<212> PRT
<213> Bos taurus

<400> 10

Asp Glu Leu Gln Asp Lys Ile His Pro Phe Ala Gln Thr Gln Ser Leu
1               5                   10                  15

Val Tyr Pro Phe
            20

```
<210>  11
<211>  29
<212>  PRT
<213>  Bos taurus

<400>  11

Asn Ile Pro Pro Leu Thr Gln Thr Pro Val Val Val Pro Pro Phe Leu
1               5                   10                  15

Gln Pro Glu Val Met Gly Val Ser Lys Val Lys Glu Ala
            20                  25


<210>  12
<211>  32
<212>  PRT
<213>  Bos taurus

<400>  12

Gly Val Ser Lys Val Lys Glu Ala Met Ala Pro Lys His Lys Glu Met
1               5                   10                  15

Pro Phe Pro Lys Tyr Pro Val Glu Pro Phe Thr Glu Ser Gln Ser Leu
            20                  25                  30


<210>  13
<211>  83
<212>  PRT
<213>  Bos taurus

<400>  13

Leu Thr Asp Val Glu Asn Leu His Leu Pro Leu Pro Leu Leu Gln Ser
1               5                   10                  15

Trp Met His Gln Pro His Gln Pro Leu Pro Pro Thr Val Met Phe Pro
            20                  25                  30

Pro Gln Ser Val Leu Ser Leu Ser Gln Ser Lys Val Leu Pro Val Pro
            35                  40                  45

Gln Lys Ala Val Pro Tyr Pro Gln Arg Asp Met Pro Ile Gln Ala Phe
    50                  55                  60

Leu Leu Tyr Gln Glu Pro Val Leu Gly Pro Val Arg Gly Pro Phe Pro
65                  70                  75                  80

Ile Ile Val
```

```
<210>  14
<211>  35
<212>  PRT
<213>  Bos taurus

<400>  14

Gln Pro Ile Arg Cys Glu Lys Asp Glu Arg Phe Phe Ser Asp Lys Ile
1               5                   10                  15

Ala Lys Tyr Ile Pro Ile Gln Tyr Val Leu Ser Arg Tyr Pro Ser Tyr
            20                  25                  30

Gly Leu Asn
        35


<210>  15
<211>  23
<212>  PRT
<213>  Bos taurus

<400>  15

Ala Leu Ile Asn Asn Gln Phe Leu Pro Tyr Pro Tyr Tyr Ala Lys Pro
1               5                   10                  15

Ala Ala Val Arg Ser Pro Ala
            20


<210>  16
<211>  20
<212>  PRT
<213>  Bos taurus

<400>  16

Ala Gly Thr Trp Tyr Ser Leu Ala Met Ala Ala Ser Asp Ile Ser Leu
1               5                   10                  15

Leu Asp Ala Gln
            20


<210>  17
<211>  35
<212>  PRT
<213>  Bos taurus

<400>  17

Arg Thr Pro Glu Val Asp Asp Glu Ala Leu Glu Lys Phe Asp Lys Ala
1               5                   10                  15

Leu Lys Ala Leu Pro Met His Ile Arg Leu Ser Phe Asn Pro Thr Gln
            20                  25                  30
```

Leu Glu Glu
35

<210>  18
<211>  20
<212>  PRT
<213>  Bos taurus

<400>  18

Leu Asn Glu Asn Leu Leu Arg Phe Phe Val Ala Pro Phe Pro Glu Val
1               5                   10                  15

Phe Gly Lys Glu
20

<210>  19
<211>  20
<212>  PRT
<213>  Bos taurus

<400>  19

Val Ala Pro Phe Pro Glu Val Phe Gly Lys Glu Lys Val Asn Glu Leu
1               5                   10                  15

Ser Lys Asp Ile
20

<210>  20
<211>  20
<212>  PRT
<213>  Bos taurus

<400>  20

Val Phe Gly Lys Glu Lys Val Asn Glu Leu Ser Lys Asp Ile Gly Ser
1               5                   10                  15

Glu Ser Thr Glu
20

<210>  21
<211>  20
<212>  PRT
<213>  Bos taurus

<400>  21

Pro Asn Ser Val Glu Gln Lys His Ile Gln Lys Glu Asp Val Pro Ser
1               5                   10                  15

Glu Arg Tyr Leu
20

```
<210>   22
<211>   20
<212>   PRT
<213>   Bos taurus

<400>   22

Gln Lys Glu Asp Val Pro Ser Glu Arg Tyr Leu Gly Tyr Leu Glu Gln
1               5                   10                  15

Leu Leu Arg Leu
            20


<210>   23
<211>   20
<212>   PRT
<213>   Bos taurus

<400>   23

Tyr Leu Gly Tyr Leu Glu Gln Leu Leu Arg Leu Lys Lys Tyr Lys Val
1               5                   10                  15

Pro Gln Leu Glu
            20


<210>   24
<211>   20
<212>   PRT
<213>   Bos taurus

<400>   24

Arg Leu Lys Lys Tyr Lys Val Pro Gln Leu Glu Ile Val Pro Asn Ser
1               5                   10                  15

Ala Glu Glu Arg
            20


<210>   25
<211>   20
<212>   PRT
<213>   Bos taurus

<400>   25

Leu Glu Ile Val Pro Asn Ser Ala Glu Glu Arg Leu His Ser Met Lys
1               5                   10                  15

Glu Gly Ile His
            20


<210>   26
```

```
<211>   20
<212>   PRT
<213>   Bos taurus

<400>   26

Ile His Ala Gln Gln Lys Glu Pro Met Ile Gly Val Asn Gln Glu Leu
1                   5                   10                  15


Ala Tyr Phe Tyr
            20


<210>   27
<211>   20
<212>   PRT
<213>   Bos taurus

<400>   27

Glu Pro Met Ile Gly Val Asn Gln Glu Leu Ala Tyr Phe Tyr Pro Glu
1                   5                   10                  15


Leu Phe Arg Gln
            20


<210>   28
<211>   20
<212>   PRT
<213>   Bos taurus

<400>   28

Ile Gly Val Asn Gln Glu Leu Ala Tyr Phe Tyr Pro Glu Leu Phe Arg
1                   5                   10                  15


Gln Phe Tyr Gln
            20


<210>   29
<211>   20
<212>   PRT
<213>   Bos taurus

<400>   29

Phe Tyr Pro Glu Leu Phe Arg Gln Phe Tyr Gln Leu Asp Ala Tyr Pro
1                   5                   10                  15


Ser Gly Ala Trp
            20


<210>   30
<211>   20
<212>   PRT
<213>   Bos taurus
```

<400> 30

```
Tyr Gln Leu Asp Ala Tyr Pro Ser Gly Ala Trp Tyr Tyr Val Pro Leu
1               5                   10                  15

Gly Thr Gln Tyr
            20
```

<210> 31
<211> 20
<212> PRT
<213> Bos taurus

<400> 31

```
Asp Ala Pro Ser Phe Ser Asp Ile Pro Asn Pro Ile Gly Ser Glu Asn
1               5                   10                  15

Ser Glu Lys Thr
            20
```

<210> 32
<211> 20
<212> PRT
<213> Bos taurus

<400> 32

```
Ser Phe Ser Asp Ile Pro Asn Pro Ile Gly Ser Glu Asn Ser Glu Lys
1               5                   10                  15

Thr Thr Met Pro
            20
```

<210> 33
<211> 20
<212> PRT
<213> Bos taurus

<400> 33

```
Thr Tyr Lys Gln Glu Lys Asn Met Ala Ile Asn Pro Ser Lys Glu Asn
1               5                   10                  15

Leu Cys Ser Thr
            20
```

<210> 34
<211> 20
<212> PRT
<213> Bos taurus

<400> 34

Asn Met Ala Ile Asn Pro Ser Lys Glu Asn Leu Cys Ser Thr Phe Cys
1               5               10              15

Lys Glu Val Val
            20

<210>   35
<211>   20
<212>   PRT
<213>   Bos taurus

<400>   35

Ser Lys Glu Asn Leu Cys Ser Thr Phe Cys Lys Glu Val Val Arg Asn
1               5               10              15

Ala Asn Glu Glu
            20

<210>   36
<211>   20
<212>   PRT
<213>   Bos taurus

<400>   36

Cys Lys Glu Val Val Arg Asn Ala Asn Glu Glu Glu Tyr Ser Ile Gly
1               5               10              15

Ser Ser Ser Glu
            20

<210>   37
<211>   20
<212>   PRT
<213>   Bos taurus

<400>   37

Ser Ala Glu Val Ala Thr Glu Glu Val Lys Ile Thr Val Asp Asp Lys
1               5               10              15

His Tyr Gln Lys
            20

<210>   38
<211>   20
<212>   PRT
<213>   Bos taurus

<400>   38

Val Ala Thr Glu Glu Val Lys Ile Thr Val Asp Asp Lys His Tyr Gln
1               5               10              15

Lys Ala Leu Asn
                20


<210>   39
<211>   20
<212>   PRT
<213>   Bos taurus

<400>   39

Glu Glu Val Lys Ile Thr Val Asp Asp Lys His Tyr Gln Lys Ala Leu
1               5                   10                  15


Asn Glu Ile Asn
                20


<210>   40
<211>   20
<212>   PRT
<213>   Bos taurus

<400>   40

Lys Ile Thr Val Asp Asp Lys His Tyr Gln Lys Ala Leu Asn Glu Ile
1               5                   10                  15


Asn Gln Phe Tyr
                20


<210>   41
<211>   20
<212>   PRT
<213>   Bos taurus

<400>   41

Leu Asn Glu Ile Asn Gln Phe Tyr Gln Lys Phe Pro Gln Tyr Leu Gln
1               5                   10                  15


Tyr Leu Tyr Gln
                20


<210>   42
<211>   20
<212>   PRT
<213>   Bos taurus

<400>   42

Gln Tyr Leu Gln Tyr Leu Tyr Gln Gly Pro Ile Val Leu Asn Pro Trp
1               5                   10                  15


Asp Gln Val Lys
                20

```
<210>   43
<211>   20
<212>   PRT
<213>   Bos taurus

<400>   43

Pro Ile Val Leu Asn Pro Trp Asp Gln Val Lys Arg Asn Ala Val Pro
1               5                   10                  15


Ile Thr Pro Thr
            20


<210>   44
<211>   20
<212>   PRT
<213>   Bos taurus

<400>   44

Thr Lys Lys Thr Lys Leu Thr Glu Glu Glu Lys Asn Arg Leu Asn Phe
1               5                   10                  15


Leu Lys Lys Ile
            20


<210>   45
<211>   20
<212>   PRT
<213>   Bos taurus

<400>   45

Thr Glu Glu Glu Lys Asn Arg Leu Asn Phe Leu Lys Lys Ile Ser Gln
1               5                   10                  15


Arg Tyr Gln Lys
            20


<210>   46
<211>   20
<212>   PRT
<213>   Bos taurus

<400>   46

Arg Glu Leu Glu Glu Leu Asn Val Pro Gly Glu Ile Val Glu Ser Leu
1               5                   10                  15


Ser Ser Ser Glu
            20


<210>   47
```

```
<211>  20
<212>  PRT
<213>  Bos taurus

<400>  47

Asp Glu Leu Gln Asp Lys Ile His Pro Phe Ala Gln Thr Gln Ser Leu
1               5                   10                  15

Val Tyr Pro Phe
            20


<210>  48
<211>  20
<212>  PRT
<213>  Bos taurus

<400>  48

Asn Ile Pro Pro Leu Thr Gln Thr Pro Val Val Val Pro Pro Phe Leu
1               5                   10                  15

Gln Pro Glu Val
            20


<210>  49
<211>  20
<212>  PRT
<213>  Bos taurus

<400>  49

Val Val Val Pro Pro Phe Leu Gln Pro Glu Val Met Gly Val Ser Lys
1               5                   10                  15

Val Lys Glu Ala
            20


<210>  50
<211>  20
<212>  PRT
<213>  Bos taurus

<400>  50

Gly Val Ser Lys Val Lys Glu Ala Met Ala Pro Lys His Lys Glu Met
1               5                   10                  15

Pro Phe Pro Lys
            20


<210>  51
<211>  20
<212>  PRT
<213>  Bos taurus
```

```
<400>  51

Glu Ala Met Ala Pro Lys His Lys Glu Met Pro Phe Pro Lys Tyr Pro
1               5                   10                  15


Val Glu Pro Phe
            20



<210>  52
<211>  20
<212>  PRT
<213>  Bos taurus

<400>  52

His Lys Glu Met Pro Phe Pro Lys Tyr Pro Val Glu Pro Phe Thr Glu
1               5                   10                  15


Ser Gln Ser Leu
            20



<210>  53
<211>  20
<212>  PRT
<213>  Bos taurus

<400>  53

Leu Thr Asp Val Glu Asn Leu His Leu Pro Leu Pro Leu Leu Gln Ser
1               5                   10                  15


Trp Met His Gln
            20



<210>  54
<211>  20
<212>  PRT
<213>  Bos taurus

<400>  54

Leu His Leu Pro Leu Pro Leu Leu Gln Ser Trp Met His Gln Pro His
1               5                   10                  15


Gln Pro Leu Pro
            20



<210>  55
<211>  20
<212>  PRT
<213>  Bos taurus

<400>  55
```

```
Leu Leu Gln Ser Trp Met His Gln Pro His Gln Pro Leu Pro Pro Thr
1               5                   10              15

Val Met Phe Pro
                20


<210>  56
<211>  20
<212>  PRT
<213>  Bos taurus

<400>  56

Ser Trp Met His Gln Pro His Gln Pro Leu Pro Pro Thr Val Met Phe
1               5                   10              15

Pro Pro Gln Ser
                20


<210>  57
<211>  20
<212>  PRT
<213>  Bos taurus

<400>  57

Leu Pro Pro Thr Val Met Phe Pro Pro Gln Ser Val Leu Ser Leu Ser
1               5                   10              15

Gln Ser Lys Val
                20


<210>  58
<211>  20
<212>  PRT
<213>  Bos taurus

<400>  58

Phe Pro Pro Gln Ser Val Leu Ser Leu Ser Gln Ser Lys Val Leu Pro
1               5                   10              15

Val Pro Gln Lys
                20


<210>  59
<211>  20
<212>  PRT
<213>  Bos taurus

<400>  59

Gln Ser Val Leu Ser Leu Ser Gln Ser Lys Val Leu Pro Val Pro Gln
1               5                   10              15
```

Lys Ala Val Pro
20

<210> 60
<211> 20
<212> PRT
<213> Bos taurus

<400> 60

Ser Gln Ser Lys Val Leu Pro Val Pro Gln Lys Ala Val Pro Tyr Pro
1               5                   10                  15

Gln Arg Asp Met
20

<210> 61
<211> 20
<212> PRT
<213> Bos taurus

<400> 61

Gln Lys Ala Val Pro Tyr Pro Gln Arg Asp Met Pro Ile Gln Ala Phe
1               5                   10                  15

Leu Leu Tyr Gln
20

<210> 62
<211> 20
<212> PRT
<213> Bos taurus

<400> 62

Pro Gln Arg Asp Met Pro Ile Gln Ala Phe Leu Leu Tyr Gln Glu Pro
1               5                   10                  15

Val Leu Gly Pro
20

<210> 63
<211> 20
<212> PRT
<213> Bos taurus

<400> 63

Phe Leu Leu Tyr Gln Glu Pro Val Leu Gly Pro Val Arg Gly Pro Phe
1               5                   10                  15

Pro Ile Ile Val
20

<210> 64
<211> 20
<212> PRT
<213> Bos taurus

<400> 64

Gln Pro Ile Arg Cys Glu Lys Asp Glu Arg Phe Phe Ser Asp Lys Ile
1               5                   10                  15

Ala Lys Tyr Ile
            20


<210> 65
<211> 20
<212> PRT
<213> Bos taurus

<400> 65

Lys Asp Glu Arg Phe Phe Ser Asp Lys Ile Ala Lys Tyr Ile Pro Ile
1               5                   10                  15

Gln Tyr Val Leu
            20


<210> 66
<211> 20
<212> PRT
<213> Bos taurus

<400> 66

Ser Asp Lys Ile Ala Lys Tyr Ile Pro Ile Gln Tyr Val Leu Ser Arg
1               5                   10                  15

Tyr Pro Ser Tyr
            20


<210> 67
<211> 20
<212> PRT
<213> Bos taurus

<400> 67

Ile Ala Lys Tyr Ile Pro Ile Gln Tyr Val Leu Ser Arg Tyr Pro Ser
1               5                   10                  15

Tyr Gly Leu Asn
            20


<210> 68

<211> 20
<212> PRT
<213> Bos taurus

<400> 68

Ala Leu Ile Asn Asn Gln Phe Leu Pro Tyr Pro Tyr Tyr Ala Lys Pro
1               5                   10                  15

Ala Ala Val Arg
            20

<210> 69
<211> 20
<212> PRT
<213> Bos taurus

<400> 69

Asn Asn Gln Phe Leu Pro Tyr Pro Tyr Tyr Ala Lys Pro Ala Ala Val
1               5                   10                  15

Arg Ser Pro Ala
            20

<210> 70
<211> 20
<212> PRT
<213> Bos taurus

<400> 70

Ala Gly Thr Trp Tyr Ser Leu Ala Met Ala Ala Ser Asp Ile Ser Leu
1               5                   10                  15

Leu Asp Ala Gln
            20

<210> 71
<211> 20
<212> PRT
<213> Bos taurus

<400> 71

Arg Thr Pro Glu Val Asp Asp Glu Ala Leu Glu Lys Phe Asp Lys Ala
1               5                   10                  15

Leu Lys Ala Leu
            20

<210> 72
<211> 20
<212> PRT
<213> Bos taurus

```
<400>   72

Glu Val Asp Asp Glu Ala Leu Glu Lys Phe Asp Lys Ala Leu Lys Ala
1               5               10                  15


Leu Pro Met His
            20


<210>   73
<211>   20
<212>   PRT
<213>   Bos taurus

<400>   73

Asp Glu Ala Leu Glu Lys Phe Asp Lys Ala Leu Lys Ala Leu Pro Met
1               5               10                  15


His Ile Arg Leu
            20


<210>   74
<211>   20
<212>   PRT
<213>   Bos taurus

<400>   74

Phe Asp Lys Ala Leu Lys Ala Leu Pro Met His Ile Arg Leu Ser Phe
1               5               10                  15


Asn Pro Thr Gln
            20


<210>   75
<211>   20
<212>   PRT
<213>   Bos taurus

<400>   75

Ala Leu Lys Ala Leu Pro Met His Ile Arg Leu Ser Phe Asn Pro Thr
1               5               10                  15


Gln Leu Glu Glu
            20
```